# FASCICULE DE BREVET EUROPEEN

(11) **EP 2 643 307 B1**
(45) Date de publication et mention de la délivrance du brevet: **17.09.2014**
(21) Numéro de dépôt: 11796759.6
(22) Date de dépôt: 18.11.2011
(51) Int. Cl.: C07D 277/68, C07D 417/12, A61K 31/428, A61P 31/00

(54) **DERIVES DE NITROBENZOTHIAZOLES ET LEUR UTILISATION POUR LE TRAITMENT DE LA TUBERCOLOSE**
NITROBENZOTHIAZOLDERIVATE UND IHRE VERWENDUNG ZUR BEHANDLUNG VON TUBERKULOSE
NITROBENZOTHIAZOLE DERIVATIVES AND USE THEREOF FOR TREATING TUBERCULOSIS

(30) Priorité: 22.11.2010 FR 1059602
(43) Date de publication de la demande: 02.10.2013
(73) Titulaire: SANOFI, 75013 Paris (FR)
(72) Inventeur: PELLET, Alain, F-75013 Paris (FR)
(74) Mandataire: Werner, Alain Henri
(86) Numéro de dépôt international: PCT/FR2011/052699
(87) Numéro de publication internationale: WO 2012/069743

(56) Documents cités:
- WO-A1-00/04901
- WO-A1-2004/067000
- DE-A1- 2 013 434
- NAYYAR A ET AL: "Recent Advances in New Structural Classes of Anti-Tuberculosis Agents", CURRENT MEDICINAL CHEMISTRY, BENTHAM SCIENCE PUBLISHERS BV, BE, vol. 12, no. 16, 1 août 2005 (2005-08-01), pages 1873-1886, XP002577805, ISSN: 0929-8673, DOI: DOI:10.2174/0929867054546654
- CHO Y ET AL: "Discovery of Novel Nitrobenzothiazole Inhibitors for Mycobacterium tuberculosis ATP Phosphoribosyl Transferase (HisG) through Virtual Screening", JOURNAL OF MEDICINAL CHEMISTRY, AMERICAN CHEMICAL SOCIETY, WASHINGTON, US, vol. 51, no. 19, 9 octobre 2008 (2008-10-09), pages 5984-5992, XP002577804, ISSN: 0022-2623, DOI: DOI:10.1021/JM800328V [extrait le 2008-09-09]

## Description

La présente invention se rapporte à des dérivés de nitrobenzothiazole, à leur préparation et à leur application en thérapeutique. Les composés selon la présente invention sont utiles notamment dans le traitement de la tuberculose.

La tuberculose est une maladie qui est aujourd'hui encore une menace pour la santé mondiale. Globalement, un tiers de la population humaine est infectée par *Mycobacterium tuberculosis.* Malgré le fait que des traitements existent et que la maladie soit curable, la tuberculose a tué approximativement 1,82 millions de personnes en 2008, et son incidence globale augmente de 1% par an, avec une estimation en 2008 de 9,4 millions de nouveaux cas par an de maladie déclarée. A cela s'ajoutent les difficultés d'une prescription correcte et d'adhésion aux protocoles de traitement, ainsi que l'émergence de souches de *M. tuberculosis* multi-résistantes. Les interactions médicament-médicament interfèrent également avec le traitement optimal du sida et de la tuberculose chez les patients co-infectés.

Les protocoles de traitement courants contre les souches de *M. tuberculosis* sensibles sont principalement basés sur une combinaison de trois, ou plus fréquemment de quatre molécules : l'isoniazide (INH), la rifampicine (RIF), la pyrazinamide (PZA) et l'éthambutol (EMB). Ces médicaments constituent la "première ligne" thérapeutique.

Durant les dernières décennies, la tuberculose est devenue résistante à chacune de ces molécules. Les souches résistantes au moins à l'isoniazide et à la rifampicine sont désignées sous le nom de "multi-résistantes" (MDR-TB). Récemment, de nouvelles souches sont apparues qui sont résistantes à un nombre plus important de molécules : celles qui sont résistantes à l'isoniazide, à la rifampicine, aux fluoroquinolones et à au moins un médicament injectable de seconde ligne sont définies en tant que "ultra-résistantes" (XDR-TB).

Selon une estimation de l'OMS effectuée en 2009, il y aurait eu 0,5 million des cas de MDR-TB en 2007. D'autres évaluations rapportent une incidence relative d'environ 11 % de souches multi-résistantes parmi tous les nouveaux cas de tuberculose.

Un autre inconvénient thérapeutique dans le traitement de la tuberculose est l'interaction de la rifampicine avec les traitements contre le VIH (Virus d'Immunodéficience Humaine), ce qui représente un obstacle dans le traitement des patients co-infectés par la tuberculose et le VIH. Les recommandations thérapeutiques actuelles contre le VIH privilégient, en première intention, une trithérapie antirétrovirale associant un inhibiteur de protéase (IP) ou un inhibiteur non nucléosidique de la transcriptase inverse (INNTI) à deux inhibiteurs nucléosidiques de la transcriptase inverse (INTI). IP et INNTI sont métabolisés par le CYP3A4. Des interactions d'ordre métabolique entre antirétroviraux (ATRV) et certains médicaments associés ont été mises en évidence. Ainsi la rifampicine, inducteur puissant du CYP3A4 intestinal et hépatique, diminue les concentrations des ATRV.

Il y a urgence à développer des thérapies améliorées contre la tuberculose. Ces nouveaux traitements contre la tuberculose doivent de préférence répondre à un ou plusieurs des critères suivants :
- raccourcir la durée du traitement pour améliorer l'adhésion aux protocoles de traitement et réduire l'apparition de bactéries résistances,
- être bien tolérées, agissant via de nouveaux mécanismes d'action et donc efficaces contre les souches multi-résistantes et/ou ultra-résistantes,
- être actifs contre la tuberculose et ne présentant ni inhibition ni induction de l'enzyme cytochrome hépatique P450, de manière à éviter des interactions médicamenteuses, en particulier avec les thérapies antirétrovirales, afin de faciliter le traitement des patients co-infectés par la tuberculose et le VIH,
- présenter un temps de traitement de la tuberculose latente (primo-infection asymptomatique) raccourcis, de manière à aborder le problème du réservoir biologique de M. *tuberculosis.*

La présente invention a en particulier pour objet des dérivés de nitrobenzothiazole, présentant une action bactériostatique ainsi que bactéricide contre des souches de *Mycobacterium* ou de *Corynebacterium* sensibles et résistantes aux antibiotiques de première ligne, leur préparation et leurs applications thérapeutiques.

Des benzothiazoles ont été décrits dans des compositions utilisées pour éloigner les insectes, les mites et les tiques (WO 99/65886) et en tant qu'inhibiteur d'adhésion cellulaire (JP2000086642).

Des benzothiazoles ont également été décrits dans la préparation de composés immunostimulants pour le traitement de maladies infectieuses, de tumeurs et de maladies immunitaires (JP2000178248).

Des nitrobenzothiazoles ont été décrits comme fongicides et bactéricides (DE 2136924) et (DE 2136923).

Des benzothiazoles pour le traitement de la tuberculose ont étés décrits dans Nayar A. et al , Curr. Med. Chem. 2005, 12, 1873 - 1886 et dans Cho y. et al., J. Med. Chem. 2008, 51, 5984 - 5992.

La présente invention a pour objet les composés répondant à la formule (I) :

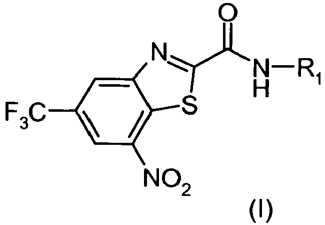

Pour lesquels, R₁ représente :
- un C₁₋₄-alkyle substitué par :
   - un ou plusieurs atomes d'halogène ;
   - un aryle éventuellement substitué par un ou plusieurs groupes choisis indépendamment les uns des autres parmi un groupe OH, C₁₋₃-alcoxy, C₃₋₆-hétérocycloalkyl, C₁₋₃-fluoroalcoxy, un atome d'halogène ;
   - un hétéroaryle éventuellement substitué par un ou plusieurs groupes choisis indépendamment les uns des autres parmi un groupe C₁₋₄-alkyle, un atome d'halogène;
   - un hétéroaryl-NH non substitué ;
   - un C₃₋₆-cycloalkyle non substitué ;
   - un C₃₋₆-hétérocycloalkyl éventuellement substitué par un ou plusieurs groupes choisis indépendamment les uns des autres parmi un groupe C₁₋₃-alkyle, aryle et aryl-C₁₋₃-alkyle ;
   ou
- un C₁₋₄-alkyle di-substitué par :
   - un aryle non substitué ;
      et
   - un C₃₋₆-hétérocycloalkyle, non substitué.

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

De par leur structure, les composés de formule générale (I) peuvent également exister sous forme d'isomères de type rotamère ou atropoisomère.

Les composés de formule (I) peuvent également exister à l'état de bases ou de sels d'addition à des acides ou à des bases. De tels sels d'addition font partie de l'invention.

Ces sels sont avantageusement préparés avec des acides ou des bases pharmaceutiquement acceptables, mais les sels d'autres acides ou bases utiles, par exemple, pour la purification ou la séparation des composés de formule générale (I) font également partie de l'invention.

Selon la présente invention, les N-oxydes des composés comportant un atome d'azote de type tertiaire ou des composés hétéroaryles azotés font également partie de l'invention à l'exception des composés de types benzothiazole-N-oxydes.

Les composés de formule (I) selon la présente invention comprennent également ceux dans lesquels un ou plusieurs atomes d'hydrogène, de carbone ou d'halogène, notamment de chlore ou de fluor ont été remplacés par leurs isotopes radioactifs, par exemple le tritium pour remplacer l'hydrogène ou le carbone 14 pour remplacer le carbone 12. De tels composés marqués sont utiles dans des travaux de recherche, de métabolisme ou de pharmacocinétique, ainsi que dans des essais biologiques et pharmacologiques en tant qu'outils.

Dans le cadre de la présente invention :
- dans Cₘ₋ₙ, les indices numériques m et n déterminent le nombre possible d'atomes de carbone présents dans une chaîne ou un cycle. Ainsi, à titre d'exemple, C₁₋₆, représentent une chaîne carbonée linéaire ou ramifiée pouvant avoir de 1 à 6 atomes de carbone, ou encore C₀₋₆, représentent une chaîne carbonée linéaire ou ramifiée pouvant avoir de 0 à 6 atomes de carbone ;
- alkyle représente un groupe aliphatique saturé, linéaire ou ramifié; par exemple, un groupe C₁₋₆-alkyle représente une chaîne carbonée de 1 à 6 atomes de carbone, linéaire ou ramifiée, notamment un méthyle, éthyle, propyle, isopropyle, butyle, isobutyle, secbutyle, *tert*butyle, pentyle ; lorsqu'un groupe alkyle est substitué par un ou plusieurs atomes d'halogène ou par un ou plusieurs groupes tels qu'indiqués dans les définitions, ces substitutions peuvent être portées par le même atome de carbone et/ou par des atomes de carbone différents ;
- cycloalkyle représente un groupe aliphatique cyclique saturé. A titre d'exemple, on peut citer les groupes cyclopropyle, cyclobutyle, cyclopentyle et cyclohexyle ;
- halogène représente un atome de fluor, de chlore, de brome ou d'iode ;
- fluoroalkyle représente un groupe alkyle dans lequel un ou plusieurs atomes d'hydrogène sont remplacés par un atome de fluor. A titre d'exemple de groupe fluoroalkyle, on peut citer le trifluorométhyle, le difluorométhyle, le 3,3,3-trifluoropropyle ;
- hétérocycloalkyle représente un cycle saturé ou partiellement saturé, monocyclique ou polycyclique éventuellement substitué, comprenant de 3 à 6 chaînons et un à plusieurs hétéroatomes tels que les atomes d'azote, d'oxygène ou de soufre. A titre d'exemple, un hétérocycloalkyle peut être une pyrrolidine, une morpholine, une pipérazine, une diazétidine, une dihydropyrrolidine, une pipéridine, un azépane, une imidazolidine, une thiomorpholine, un tétrahydropyrane, un tétrahydrothiopyrane, une pipérazine, une diazépane ou une azabicyclooctane, un tropane, un 3,6-diaza-bicyclo[3.1.0]hexane ;
- aryle représente un système aromatique monocyclique ou polycyclique éventuellement substitué, comprenant de 6 à 14 atomes de carbone, de préférence de 6 à 10 atomes de carbone. Lorsque le système est polycyclique au moins un des cycles est aromatique. A titre d'exemples de groupes aryles, on peut citer le phényle, le naphtyle, l'indanyle, le tétrahydronaphtyle, l'anthracènyle ou l'azulènyle ;
- hétéroaryle représente un système aromatique monocyclique ou polycyclique éventuellement substitué comprenant de 5 à 14 chaînons, de préférence de 5 à 10 chaînons et comprenant un à plusieurs hétéroatomes tels que les atomes d'azote, d'oxygène ou de soufre. Lorsque le système est polycyclique au moins un des cycles est aromatique. Les atomes d'azote peuvent être sous forme de N-oxydes. A titre d'exemple d'hétéroaryles monocycliques on peut citer le thiazole, le thiadiazole, le thiophène, l'imidazole, le triazole, le tétrazole, la pyridine, le furane, l'oxazole, l'isoxazole, l'oxadiazole, le pyrrole, le pyrazole, la pyrimidine, la pyridazine et la pyrazine. A titre d'exemple d'hétéroaryles polycycliques, on peut citer l'indole, le benzofurane, le benzimidazole, le benzothiophène, le benzotriazole, le benzothiazole, la benzoxazole, la quinoline, l'isoquinoline, l'indazole, la quinazoline, la phthalazine, la quinoxaline, la naphtyridine, le 2,3-dihydro-1H-indole, le 2,3-dihydro-benzofurane, la tétrahydroquinoline, la tétrahydroisoquinoline ou la tétrahydroisoquinazoline ;
- alcoxy représente un groupe -O-Cₘ₋ₙ-alkyle à chaîne aliphatique saturée, linéaire ou ramifiée. A titre d'exemple de groupes alcoxy, on peut citer le méthoxy ;
- fluoroalcoxy représente un groupe alcoxy dans lequel un ou plusieurs atomes d'hydrogène sont remplacés par un atome de fluor. A titre d'exemples de groupe fluoroalcoxy, on peut citer les groupes -O-CHF₂, -O-CF₃, ou -O-CF₂-CF₃ ;
- aryloxy représente un groupe -O-aryle ;
- hétéroaryloxy représente un groupe -O-hétéroaryle ;
- hydroxyalkyle représente un groupe -C₁₋₆-alkyle-OH. A titre d'exemple de groupe hydroxyalkyle, on peut citer le groupe -CH₂-OH ;

Selon la présente invention, on préfère les composés de formule (I) dans lesquels R₁ représente :
- un radical C₁₋₄-alkyle substitué par :
   - un ou plusieurs atomes de fluor ;
   - un phényle éventuellement substitué par un ou plusieurs groupes choisis indépendamment les uns des autres parmi un groupe OH, méthoxy, morpholine, trifluorométhoxy, un atome de chlore ou de fluor ;
   - un groupe pyridinyle, isoxazolyle, pyrazolyle, thiényle, benzoxazolyle, tétrahydroisoquinoléinyle, quinoléinyle et thiazolyle éventuellement substitué par un ou plusieurs groupes choisis indépendamment les uns des autres parmi un groupe méthyle, éthyle ou un atome de chlore ;
   - un groupe pyridinyl-NH non substitué ;
   - un groupe cyclohexyle non substitué ;
   - un groupe tétrahydropyranyle, un groupe morpholinyle, un groupe pyrrolidinyle et thiomorpholinyle éventuellement substitué par un ou plusieurs groupes choisis indépendamment les uns des autres parmi un groupe méthyle, phényle et benzyle ;
   ou
- un radical C₁₋₄-alkyle di-substitué par :
   - un phényle non substitué ;
      et
   - un groupe morpholinyle non substitué.

Parmi les composés selon l'invention, on peut notamment citer les composés ci-après, tels quels ainsi que leurs sels :

| **Structure Chimique** | **Nom IUPAC (ACDName)** |
|---|---|
| 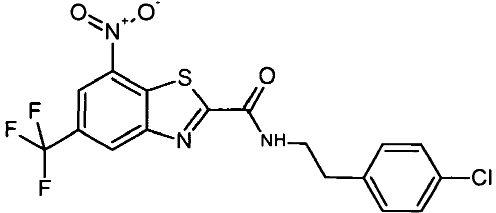 | *N*-[2-(4-chlorophényl)éthyl]-7-nitro-5-(trifluorométhyl)-1,3-benzothiazole-2-carboxamide |
| 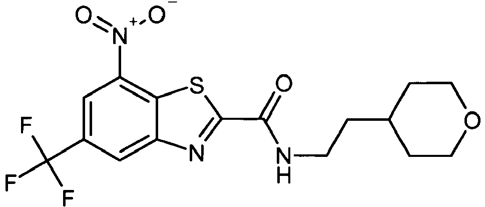 | 7-nitro-*N*-[2-(tétrahydro-2*H*-pyran-4-yl)éthyl]-5-(trifluorométhyl)-1,3-benzothiazole-2-carboxamide |
| 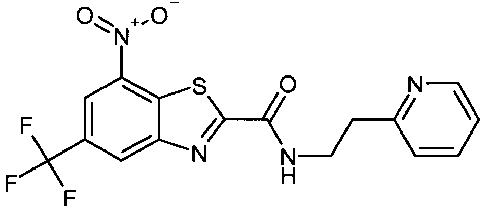 | 7-nitro-*N*-(2-pyridin-2-yléthyl)-5-(trifluorométhyl)-1,3-benzothiazole-2-carboxamide |
| 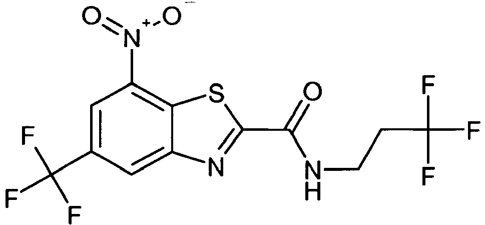 | 7-nitro-5-(trifluorométhyl)-*N*-(3,3,3-trifluoropropyl)-2-benzothiazolecarboxamide |
| 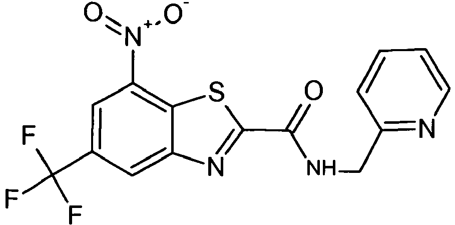 | 7-nitro-*N*-(pyridin-2-ylméthyl)-5-(trifluorométhyl)-1,3-benzothiazole-2-carboxamide |
| 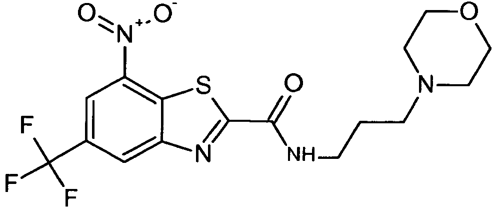 | *N*-[3-(4-morpholinyl)propyl]-7-nitro-5-(trifluorométhyl)- 2-benzothiazolecarboxamide |
| 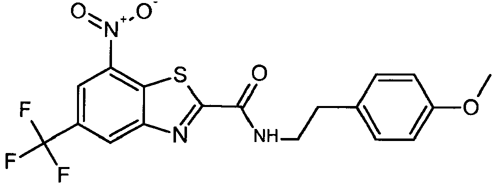 | *N*-[2-(4-méthoxyphényl)éthyl]-7-nitro-5-(trifluorométhyl)-1,3-benzothiazole-2-carboxamide |
| 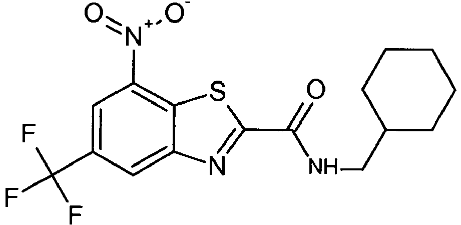 | *N*-(cyclohexylméthyl)-7-nitro-5-(trifluorométhyl)-1,3-benzothiazole-2-carboxamide |
| 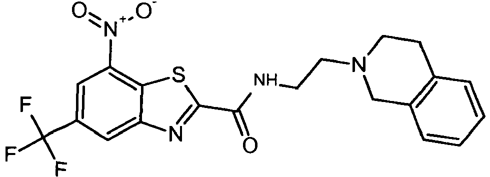 | *N*-[2-(1,2,3,4-tétrahydroisoquinoléin-2(1*H*)yl)éthyl]-7-nitro-5-(trifluorométhyly)-1,3-benzothiazole-2-carboxamide |
| 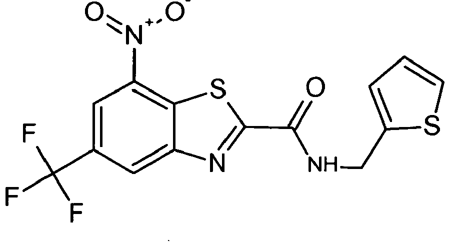 | 7-nitro-*N*-(thién-2-ylméthyl)-5-(trifluorométhyl)-1,3-benzothiazole-2-carboxamide |
| 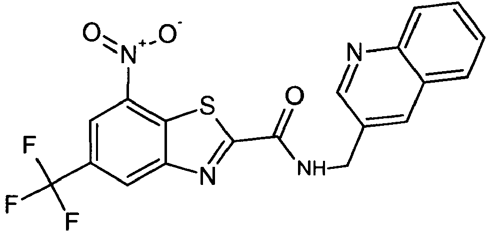 | 7-nitro-*N*-(quinoléin-3-ylméthyl)-5-(trifluorométhyl)-1,3-benzothiazole-2-carboxamide |
| 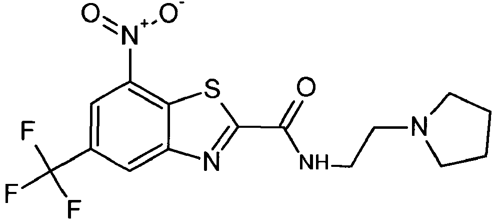 | 7-nitro-*N*-(2-pyrrolidin-1-yléthyl)-5-(trifluorométhyl)-1,3-benzothiazole-2-carboxamide |
| 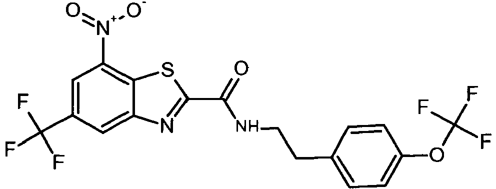 | 7-nitro-*N-*{2-[4-(trifluorométhoxy)phényl]éthyl}-5-(trifluorométhyl)-1,3-benzothiazole-2-carboxamide |
| 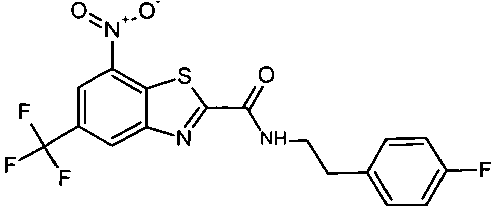 | *N*-[2-(4-fluorophényl)éthyl]-7-nitro-5-(trifluorométhyl)-1,3-benzothiazole-2-carboxamide |
| 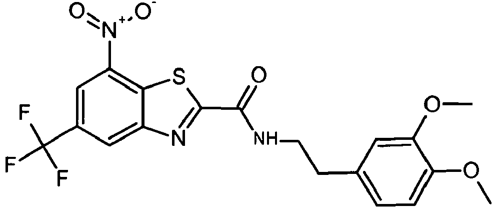 | *N-*[2-(3,4-diméthoxyphényl)éthyl]-7-nitro-5-(trifluorométhyl)-1,3-benzothiazole-2-carboxamide |
| 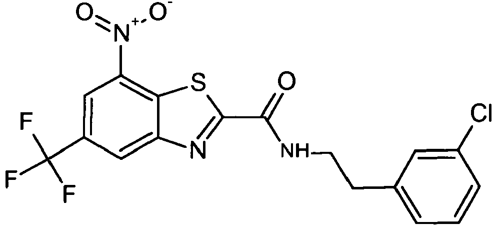 | *N*-[2-(3-chlorophényl)éthyl]-7-nitro-5-(trifluorométhyl)-1,3-benzothiazole-2-carboxamide |
| 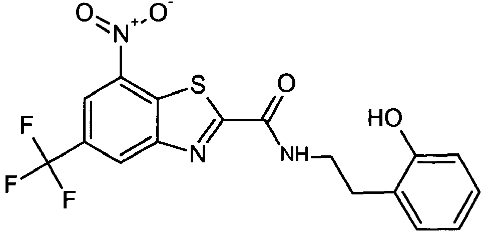 | *N*-[2-(2-hydroxyphényl)éthyl]-7-nitro-5-(trifluorométhyl)-1,3-benzothiazole-2-carboxamide |
| 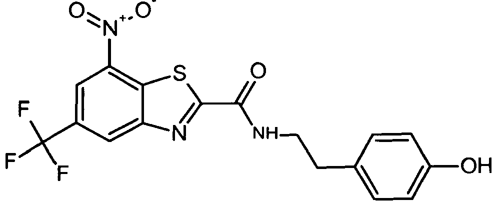 | *N*-[2-(4-hydroxyphényl)éthyl]-7-nitro-5-(trifluorométhyl)-1,3-benzothiazole-2-carboxamide |
| 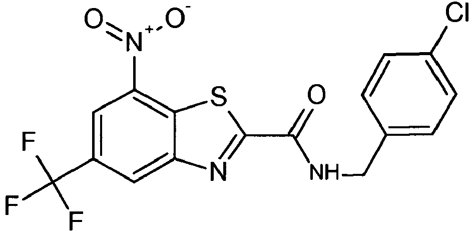 | *N*-(4-chlorobenzyl)-7-nitro-5-(trifluorométhyl)-1,3-benzothiazole-2-carboxamide |
| 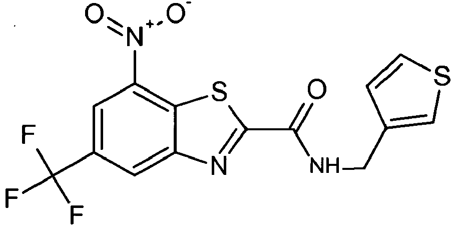 | 7-nitro-*N*-(thién-3-ylméthyl)-5-(trifluorométhyl)-1,3-benzothiazole-2-carboxamide |
| 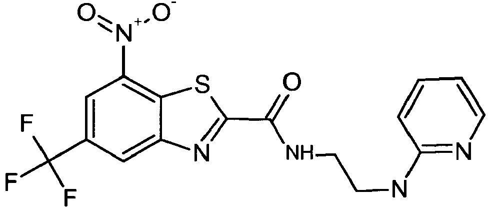 | 7-nitro-*N*-[2-(pyridin-2-ylamino)éthyl]-5-(trifluorométhyl)-1,3-benzothiazole-2-carboxamide |
| 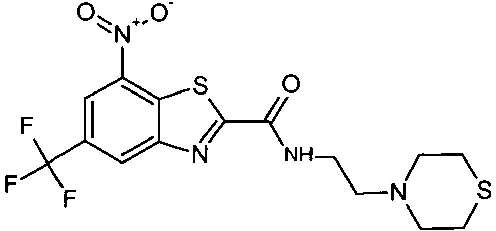 | 7-nitro-*N-*(2-thiomorpholin-4-yléthyl)-5-(trifluorométhyl)-1,3-benzothiazole-2-carboxamide |
| 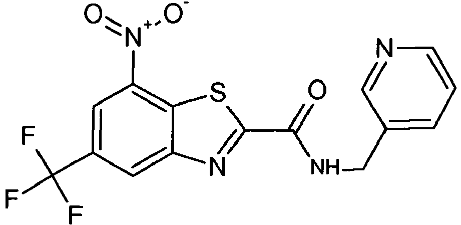 | 7-nitro-*N-*(pyridin-3-ylméthyl)-5-(trifluorométhyl)-1,3-benzothiazole-2-carboxamide |
| 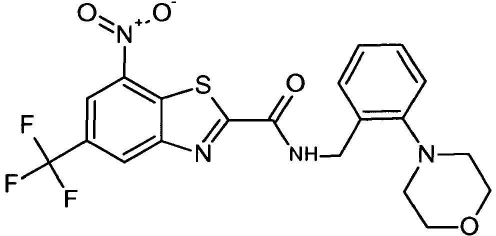 | *N*-(2-morpholin-4-ylbenzyl)-7-nitro-5-(trifluorométhyl)-1,3-benzothiazole-2-carboxamide |
| 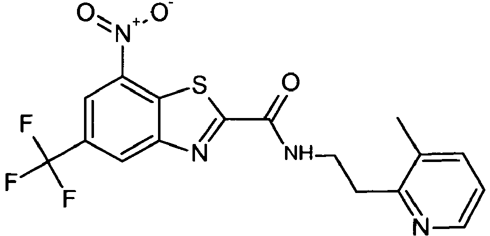 | *N*-[2-(3-méthylpyridin-2-yl)éthyl]-7-nitro-5-(trifluorométhyl)-1,3-benzothiazole-2-carboxamide |
| 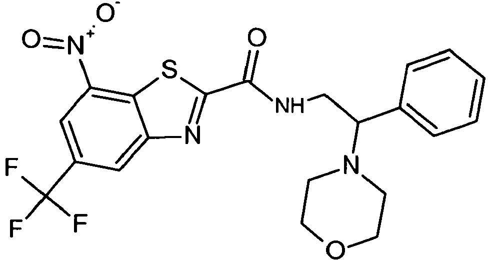 | *N*-(2-morpholin-4-yl-2-phényléthyl)-7-nitro-5-(trifluorométhyl)-1,3-benzothiazole-2-carboxamide |
| 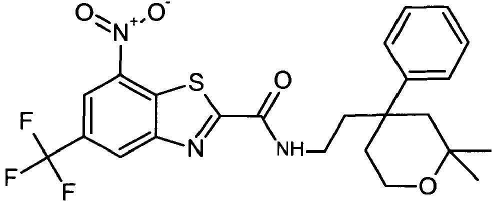 | *N*-[2-(2,2-diméthyl-4-phényltétrahydro-2*H*-pyran-4-yl)éthyl]-7-nitro-5-(trifluorométhyl)-1,3-benzothiazole-2-carboxamide |
| 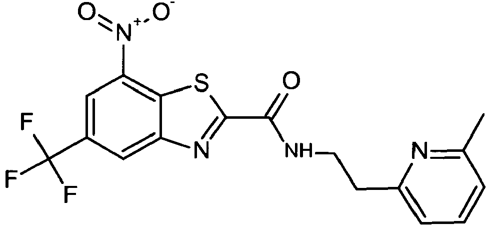 | *N*-[2-(6-méthylpyridin-2-yl)éthyl]-7-nitro-5-(trifluorométhyl)-1,3-benzothiazole-2-carboxam ide |
| 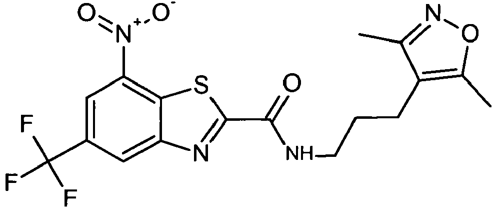 | *N*-[3-(3,5-diméthylisoxazol-4-yl)propyl]-7-nitro-5-(trifluorométhyl)-1,3-benzothiazole-2-carboxamide |
| 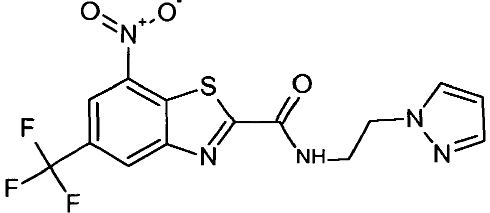 | 7-nitro-*N*-[2-(1*H*-pyrazol-1-yl)éthyl]-5-(trifluorométhyl)-1,3-benzothiazole-2-carboxamide |
| 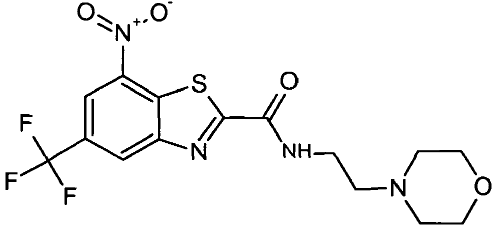 | *N*-(2-morpholin-4-yléthyl)-7-nitro-5-(trifluorométhyl)-1,3-benzothiazole-2-carboxamide |
| 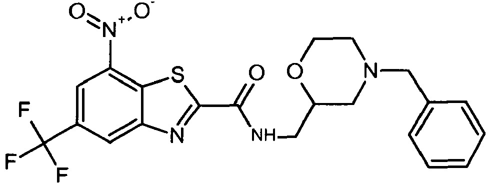 | *N*-[(4-benzylmorpholin-2-yl)méthyl]-7-nitro-5-(trifluorométhyl)-1,3-benzothiazole-2-carboxamide |
| 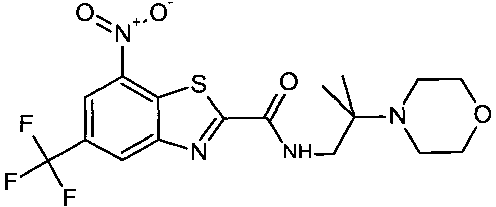 | *N*-(2-méthyl-2-morpholin-4-ylpropyl)-7-nitro-5-(trifluorométhyl)-1,3-benzothiazole-2-carboxamide |
| 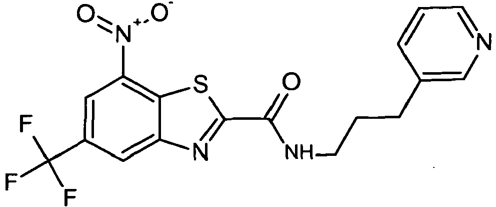 | 7-nitro-*N*-(3-pyridin-3-ylpropyl)-5-(trifluorométhyl)-1,3-benzothiazole-2-carboxamide |
| 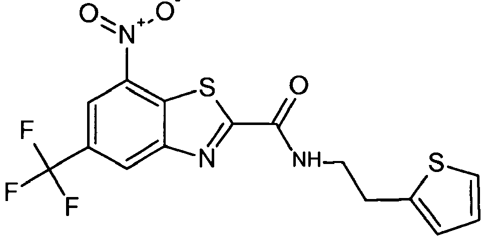 | 7-nitro-*N-*(2-thién-2-yléthyl)-5-(trifluorométhyl)-1,3-benzothiazole-2-carboxamide |
| 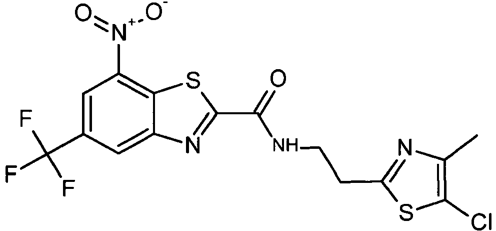 | N-[2-(5-chloro-4-méthyl-1,3-thiazol-2-yl)éthyl]-7-nitro-5-(trifluorométhyl)-1,3-benzothiazole-2-carboxamide |
| 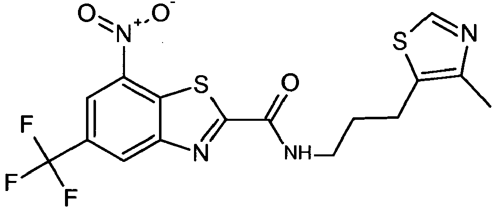 | *N*-[3-(4-méthyl-1,3-thiazol-5-yl)propyl]-7-nitro-5-(trifluorométhyl)-1,3-benzothiazole-2-carboxamide |
| 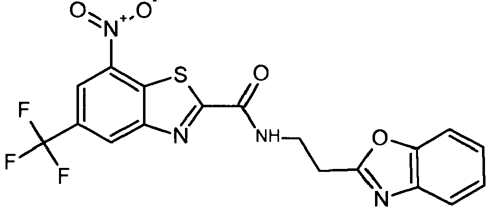 | *N*-[2-(1,3-benzoxazol-2-yl)éthyl]-7-nitro-5-(trifluorométhyl)-1,3-benzoth iazole-2-carboxam ide |
| 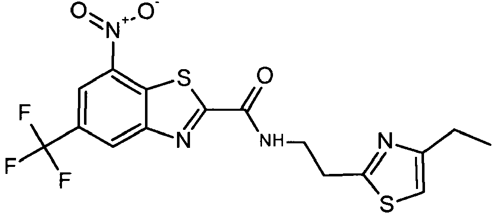 | *N*-[2-(4-éthyl-1,3-thiazol-2-yl)éthyl]-7-nitro-5-(trifluorométhyl)-1,3-benzothiazole-2-carboxamide |
| 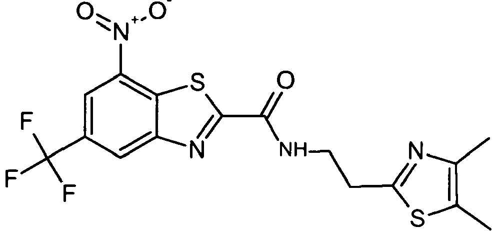 | *N*-[2-(4,5-diméthyl-1,3-thiazol-2-yl)éthyl]-7-nitro-5-(trifluorométhyl)-1,3-benzothiazole-2-carboxamide |

On préfère tout particulièrement les composés suivants ainsi que leurs sels :
- *N*-[2-(4-chlorophényl)éthyl]-7-nitro-5-(trifluorométhyl)-1,3-benzothiazole-2-carboxamide ;
- 7-nitro-N-[2-(tétrahydro-2*H*-pyran-4-yl)éthyl]-5-(trifluorométhyl)-1,3-benzothiazole-2-carboxamide ;
- N-[2-(4-méthoxyphényl)éthyl]-7-nitro-5-(trifluorométhyl)-1,3-benzothiazole-2-carboxamide ;
- *N*-[2-(1,2,3,4-tétrahydrosoquinoléin-2(1*H*)-yl)éthyl]-7-nitro-5-(trifluorométhyl)-1,3-benzothiazole-2-carboxamide ;
- *N*-[2-(4-fluorophényl)éthyl]-7-nitro-5-(trifluorométhyl)-1,3-benzothiazole-2-carboxamide ;
- *N*-[2-(3,4-diméthoxyphényl)éthyl]-7-nitro-5-(trifluorométhyl)-1,3-benzothiazole-2-carboxamide ;
- *N*-[2-(3-chlorophényl)éthyl]-7-nitro-5-(trifluorométhyl)-1,3-benzothiazole-2-carboxamide ;
- *N*-[2-(2-hydroxyphényl)éthyl]-7-nitro-5-(trifluorométhyl)-1,3-benzothiazole-2-carboxamide ;
- N-[2-(4-hydroxyphényl)éthyl]-7-nitro-5-(trifluorométhyl)-1,3-benzothiazole-2-carboxamide ;
- 7-nitro-N-[2-(pyridin-2-ylamino)éthyl]-5-(trifluorométhyl)-1,3-benzothiazole-2-carboxamide ;
- 7-nitro-*N*-(2-thiomorpholin-4-yléthyl)-5-(trifluorométhyl)-1,3-benzothiazole-2-carboxamide ;
- *N*-[2-(3-méthylpyridin-2-yl)éthyl]-7-nitro-5-(trifluorométhyl)-1,3-benzothiazole-2-carboxamide ;
- *N*-(2-morpholin-4-yl-2-phényléthyl)-7-nitro-5-(trifluorométhyl)-1,3-benzothiazole-2-carboxamide ;
- *N*-[2-(2,2-diméthyl-4-phényltétrahydro-2*H*-pyran-4-yl)éthyl]-7-nitro-5-(trifluorométhyl)-1,3-benzothiazole-2-carboxamide ;
- *N*-[2-(5-chloro-4-méthyl-1,3-thiazol-2-yl)éthyl]-7-nitro-5-(trifluorométhyl)-1,3-benzothiazole-2-carboxamide ;
- *N*-[2-(1,3-benzoxazol-2-yl)éthyl]-7-nitro-5-(trifluorométhyl)-1,3-benzothiazole-2-carboxamide ;
- *N*-[2-(4-éthyl-1,3-thiazol-2-yl)éthyl]-7-nitro-5-(trifluorométhyl)-1,3-benzothiazole-2-carboxamide ;
- *N*-[2-(4,5-diméthyl-1,3-thiazol-2-yl)éthyl]-7-nitro-5-(trifluorométhyl)-1,3-benzothiazole-2-carboxamide.

Dans ce qui suit, on entend par groupe protecteur PG un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Group in Organic Synthesis », Green et al., 4th Edition, John Wiley & Sons, Inc., New York, 2007.

Dans ce qui suit, on entend par GP un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advanced Organic Chemistry », M. B. Smith and J. March, 6th Edition, Wiley Interscience, 2007, p.496-501.

Conformément à l'invention, on peut préparer les composés de formule générale (I) selon le procédé **(voie A)** caractérisé en ce que l'on fait réagir un composé de formule (II) : 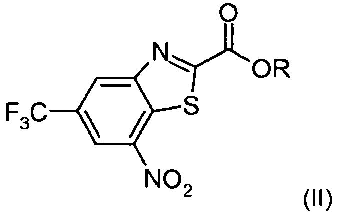 dans laquelle R représente un C₁₋₄-alkyle, avec une amine de formule (III) :

H₂N-R₁ (III)

dans laquelle R₁ est tel que défini pour les composés de formule (I).

La réaction s'effectue éventuellement en présence d'une base minérale ou organique dans le cas où l'amine secondaire de formule (III) est sous forme de sel d'acide (par exemple de chlorhydrate) dans un solvant polaire tel qu'un alcool, le DMF ou le DMSO et à une température comprise entre 5°C et 100°C.

La réaction s'effectue de préférence dans le MeOH à TA avec éventuellement de la triéthylamine.

Selon une variante **(voie B),** on peut préparer les composés de formule (I) selon le procédé caractérisé en ce que on effectue une réaction de réduction des composés de formule (IV) présentant un noyau benzothiazole N-oxyde : 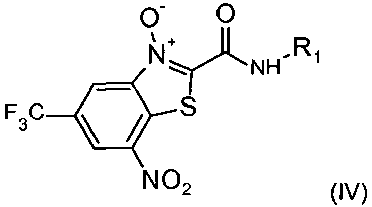 dans laquelle R₁ est tel que défini pour les composés de formule (I).

La réaction s'effectue de préférence à l'aide d'un réactif phosphoré trivalent comme le phosphite de triéthyle dans un alcool comme l'éthanol (Wagner et al, Chem. Ber. 1973, 106, 640-654).

Les composés de formule (II) dans lesquels R représente un C₁₋₄-alkyle sont obtenus par réduction des dérivés N-oxyde correspondants de formule (V) : 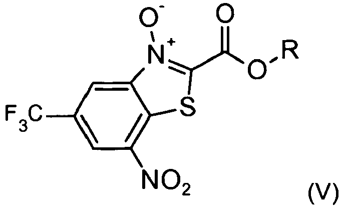 dans laquelle R représente un C₁₋₄-alkyle.

La réaction s'effectue à l'aide d'un réactif phosphoré trivalent comme le phosphite de triéthyle, dans un alcool comme l'éthanol (Wagner et al, Chem. Ber. 1973, 106, 640-654).

Les composés de formule (III) sont disponibles dans le commerce ou décrits dans la littérature, ou bien peuvent être préparés selon des méthodes qui y sont décrites ou qui sont connues de l'Homme du Métier.

Les composés de formule (IV) présentant un noyau benzothiazole N-oxyde dans laquelle R₁ est tel que défini pour les composés de formule (I) sont obtenus par réaction des dérivés N-oxyde correspondants de formule (V) dans laquelle R représente un C₁₋₄alkyle avec une amine de formule (III) dans laquelle R₁ est tel que défini pour les composés de formule (I).

La réaction s'effectue entre 5 et 100°C, dans un solvant polaire tel qu'un alcool, la DMF ou le DMSO, éventuellement en présence d'une base minérale ou organique dans le cas où l'amine secondaire de formule (III) est sous forme de sel d'acide (par exemple de chlorhydrate). La réaction s'effectue de préférence dans le MeOH à TA en présence éventuellement de triéthylamine.

Les composés de formule (V) dans laquelle R représente un C₁₋₄alkyle sont connus ou décrits (par exemple dans Wagner et al, Chem. Ber. 1973, 106, 640-654) ou bien préparés par réaction d'un ester de l'acide thioglycolique de formule (VI) : 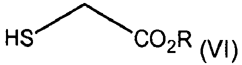 dans laquelle R représente un C₁₋₄-alkyle avec un composé de formule (VII) : 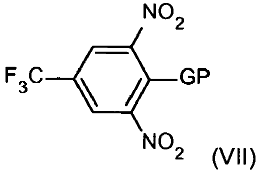 dans laquelle GP représente un groupe partant, de préférence un halogène.

Les composés de formule (VI) et (VII) sont commerciaux ou préparés selon les méthodes décrites dans la littérature.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau I ci-après, qui illustre les structures chimiques et les propriétés physiques de quelques composés selon l'invention.

Les points de fusion (PF) sont exprimés en degré Celsius. Ils ont été mesurés soit avec un appareil de Köffler (mention (K) dans ce qui suit), soit avec un appareil Mettler-Toledo FP62 (mention (M) dans ce qui suit).

Les réactifs chimiques commerciaux ainsi que les solvants de réaction ou de chromatographie sont utilisés sans purification ou distillation préalable.

Les réactions sont suivies par chromatographie sur couche mince (gel de silice 60F254, Merck) avec une détection UV à 254nM ou par pulvérisation d'une solution de réactif de Draggendorff/AcOH/H₂O/EtOH : 2/3/10/10 ou par pulvérisation d'une solution de ninhydrine 0.5% / n-butanol (Prolabo) suivie d'un chauffage à ~100°C.

D'une manière générale, les solvants sont éliminés à l'évaporateur rotatif sous pression réduite à 40°C.

Les purifications par chromatographie sont réalisées sur Combiflash ISCO en utilisant des cartouches de silice pré-conditionnées Redisep ou Chromabond Flash RS 6 SiOH ou par HPLC automatisée (Autosampler Waters 2767, pompe Waters 2525, détecteur MS Waters ZQ) sur colonne Waters sunfire C18 (30x100 mm) PDA : waters 2926.

Les analyses LCMS sont réalisées :
- sur une chaîne HPLC Waters 1525 (détecteur UV MUX 2488, 220nm) couplée à un spectromètre de masse Waters LCT, ionisation par MUX 8 mode positif ESI⁺ (Méthode A) ou mode négatif ESI⁻ (Méthode B) ;
- sur une chaîne HPLC Agilent série 1100 (détection UV à 220nm) couplée à un spectromètre de masse MSD SL (Agilent), ionisation par Electrospray mode positif ESI⁺, logiciel : Chemstation version B.01.03 de Agilent (Méthode C).

| Méthode | **A** | **B** | **C** |
|---|---|---|---|
| colonne | Waters UPLC BEH C18 50 × 2,1 mm ; 1,7m 55°C | Waters UPLC BEH C18 50 × 2,1mm ; 1,7µm 55°C | C18 Symetry 50 × 2,1 mm ; 3,54µm |
| solvants | MeCN+0,08%TFA: H₂O+0,1%TFA | MeCN+0,08%TFA: H₂O+0,1%TFA | MeCN+0,005%TFA: H₂O+0,005%TFA |
| gradiant | 5: 95 à 95: 5 (1,1min) à 95 : 5 (1,7min) | 5 : 95 à 95 : 5 (1,1min) à 95: 5 (1,7min) | 0:100 à 100 : 0 (10min) à 100 : 0 (5min) |
| débit | 0,9mL/min | 0,9mUmin | 0,4mL/min |
| ionisation | ESI⁺ | ESI⁻ | ESI⁺ |

Les caractéristiques analytiques LCMS des produits sont le rapport m/z de l'ion (M⁺H)⁺ ou de l'ion (M⁻H)⁻ et le temps de rétention (tr) du pic correspondant, observé en UV le plus souvent à 220nm et exprimé en minutes.

Les spectres de résonnance magnétique nucléaire du proton (RMN¹H) ont été effectués à 300 MHz, 400 MHz ou 500 MHz sur des appareils Brüker. Les abréviations utilisées pour caractériser les signaux sont les suivantes: s = singulet, m = multiplet, d = doublet, t = triplet, q = quadruplet.

Les abréviations et symboles utilisés pour la description des modes opératoires de synthèse et pour la description des composés sont les suivants :
- DMF pour diméthylformamide,
- DMSO pour diméthylsulfoxyde,
- THF pour tétrahydrofurane,
- HCl pour acide chlorhydrique,
- HBr pour acide bromhydrique,
- NaOH pour soude,
- Et pour éthyle,
- Me pour méthyle,
- TFA pour acide trifluoroacétique,
- TA pour température ambiante,
- NMP pour N-méthyl pyrrolidinone,
- SPR pour synthèse parallèle robotisée,
- BOC pour *tert*-butyloxy-carbonyle,
- LCMS pour "liquid chromatography coupled with mass spectrometry.

### Préparation du composé n° 1 (Exemple 1 - Voie B)

### N-[2-(4-chlorophényl)éthyl]-7-nitro-5-(trifluorométhyl)-1,3-benzothiazole-2-carboxamide.

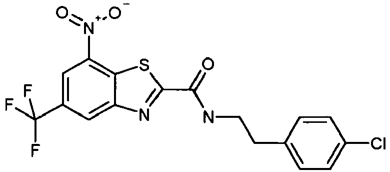

### 1.1. N-[2-(4-chlorophényl)éthyl]-7-nitro-5-(trifluorométhyl)-1,3-benzothiazole-2-carboxamide-3-oxyde.

Une suspension de 0,3g (0,89 mmol) de 7-nitro-5-(trifluorométhyl)-1,3-benzothiazole-2-carboxylate-3-oxyde d'éthyle (dont la synthèse est décrite dans Wagner et all, Chem.Ber., 1973, 106, 640-654 composé 4e et le brevet Bayer DE 2013434 exemple 7), dans 9 mL de méthanol est chauffée sous agitation jusqu'à solubilisation puis refroidie à 25°C. La solution obtenue est additionnée lentement de 0,157g (1,01 mmol) de 2-(4-chlorophényl)éthanamine. Le mélange réactionnel homogène est agité à TA pendant une nuit. Il est ensuite concentré sous pression réduite et le résidu obtenu est purifié par chromatographie sur colonne de silice (solvant: cyclohexane/dichlorométhane de 60/40 à 0/100). On obtient 0,240g de composé attendu.
LCMS : (M⁺H)⁺ = 446 ; tr =9,10 min (Méthode C).

### 1.2. N-[2-(4-chlorophényl)éthyl]-7-nitro-5-(trifluorométhyl)-1,3-benzothiazole-2-carboxamide.

Une suspension de 0,5 g (1,12 mmol) de composé obtenu à l'étape précédente dans 7 mL d'éthanol est additionnée, goutte à goutte, à 0,22 mL (1,26 mmol) de phosphite de triéthyle. Le mélange réactionnel est ensuite agité à 70°C pendant 2 heures puis refroidi à TA. Le précipité obtenu est rincé avec de l'éther de pétrole. Après séchage à 55°C sous pression réduite, on obtient 0,407g de composé attendu.
Point de fusion : 140°C (M) ; LCMS : (M⁺H)⁺ = 430 ; tr = 10,16 minutes (Méthode C).

### Préparation des composés n° 2 à 13 (Voie A)

### Exemple 2 - composé n° 2

### 7-nitro-N-(2-(tétrahydro-2H-pyran-4-yl)éthyl)-5-(trifluorométhyl)-1,3-benzothiazole-2-carboxamide.

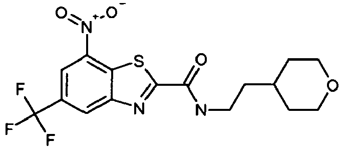

### 2.1. 7-nitro-5-(trifluorométhyl)-1,3-benzothiazole-2-carboxylate d'éthyle.

Une suspension de 20 g (59,48 mmol) de 7-nitro-5-(trifluorométhyl)-1,3-benzothiazole-2-carboxylate-3-oxyde d'éthyle, dont la synthèse est réalisée selon un procédé déjà décrit dans la littérature (K.Wagner et al ; Chem.Ber. 106, 640-654, 1973), dans 300 mL d'éthanol est agitée à TA. Après addition, goutte à goutte, de 11,22 mL (65,43 mmol) de phosphite de triéthyle, le mélange réactionnel est agité à 70°C pendant 2 heures durant lesquelles il devient homogène et jaune. Il est ensuite additionné de silice et le solvant est évaporé sous pression réduite. La poudre obtenue est déposée sur une colonne de silice. La purification est effectuée à l'aide d'un mélange cyclohexane/acétate d'éthyle. Les fractions de produit pur sont évaporées et le résidu est repris avec de l'éther de pétrole, filtré puis rincé avec un minimum d'éther éthylique. Après séchage à 40°C, sous pression réduite, obtient 16,98 g de composé attendu.
RMN ¹H (DMSO-d6, 400 MHz) δ ppm : 1,42 (t, 3H) ; 4,51 (d, 2H) ; 8,83 (s, 1H) ; 9,23 (s, 1 H) ; MS : (M⁺H)⁺ = 321.

### 2.2. 7-nitro-N-[2-(tétrahydro-2H-pyran-4-yl)éthyl]-5-(trifluorométhyl)-1,3-benzothiazole-2-carboxamide.

Une solution de 0,1 g (0,31 mmol) du composé obtenu à l'étape précédente dans 3,1 mL de méthanol est additionnée de 0,081 g (0,62 mmol) de 2-(tétrahydro-2H-pyran-4-yl)éthanamine. Le mélange réactionnel est agité à TA pendant 18 heures. Le solvant est alors évaporé sous pression réduite et le résidu obtenu est purifié par chromatographie sur silice (éluant : dichlorométhane/méthanol de 100/0 à 90/10). On obtient 0,081 g de composé attendu.
Point de fusion : 154°C (K) ; LCMS : (M⁺H)⁺ = 404 ; tr = 8,77 minutes (Méthode C).

### Préparation des composés n° 14 à 40 (Voie A - synthèse parallèle robotisée)

### Exemple 3 - composé n°16 : N-[2-(3-chlorophényl)éthyl]-7-nitro-5-(trifluorométhyl)-1,3-benzothiazole-2-carboxamide.

Dans un tube en verre, 0,94 g (0,6 mmol) de 2-(3-chlorophényl)éthanamine sont mis en solution dans 4 mL de méthanol. Le mélange est ensuite additionné de 0,16 g (0,5 mmol) du composé de la préparation 2.1. en solution dans 1mL de DMF. Le mélange réactionnel est agité à température ambiante pendant une nuit. Le méthanol est évaporé et le volume de résidu est ajusté à 4mL par de la DMF. La solution obtenue est purifiée par HPLC préparative en phase inverse selon les conditions suivantes :
- Colonne : "Xbridge Prep Phenyl 5µm OBD, Waters, 50 mm x 50 mm"
- Eluant : eau avec 0.1 % de TFA/méthanol de 75/25 à 2/98
- Débit : 120 mL/min

Le produit attendu est obtenu sous forme de trifluoroacétate.
LCMS : (M⁺H)⁻ = 428 ; tr = 1,29 minutes (Méthode B)

Le tableau I qui suit illustre les structures chimiques et les propriétés physiques de quelques exemples de composés selon l'invention.

Dans la colonne "Sel / Ion / tr (mn) / Conditions / Point de fusion" sont regroupées des informations concernant l'état des composés (sel ou base), leur caractéristiques LCMS et leur point de fusion lorsque celui-ci a été mesuré. La première ligne ("Sel") de chaque case représente l'état du composé: "/" pour un composé sous forme de base libre et "TFA" pour un composé sous forme de sel d'acide trifluoroacétique. La seconde et la troisième ligne de chaque case indiquent les caractéristiques LCMS avec entre parenthèses les méthodes A, B ou C utilisées telles que décrites précédemment. La quatrième ligne indique le point de fusion avec entre parenthèses la lettre symbolisant l'appareil de mesure : K pour le banc de Koffler et M pour l'appareil Mettler-Toledo FP62.

**TABLEAU I**

| | | | |
|---|---|---|---|
| 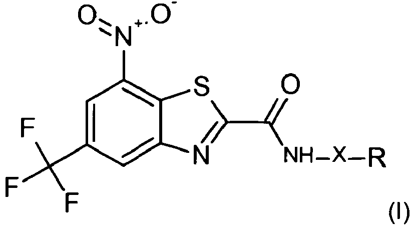 | | | |

| **Composés No.** | **X** | **R** | **Sel** |
|---|---|---|---|
| | | | **Ion** |
| | | | **tr (mn) (Conditions)** |
| | | | **Point de fusion** |
| 1 | -(CH₂)₂ | 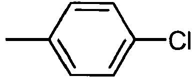 | / |
| | | | (M⁺H)⁺ = 430 |
| | | | 10,16 (C) |
| | | | 140°C (M) |
| 2 | -(CH₂)₂ | 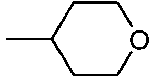 | / |
| | | | (M⁺H)⁺ = 404 |
| | | | 8,77 (C) |
| | | | 154°C (K) |
| 3 | -(CH₂)₂ | 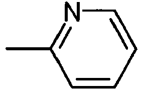 | / |
| | | | (M⁺H)⁺ 397 |
| | | | 6,3 (C) |
| | | | 146°C (K) |
| 4 | -(CH₂)₂ | 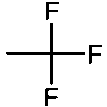 | / |
| | | | (M⁺H)⁺ = 388 |
| | | | 9,22 (C) |
| | | | 110°C (K) |
| 5 | -(CH₂) | 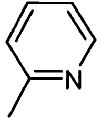 | / |
| | | | (M⁺H)⁺ = 383 |
| | | | 7,58 (C) |
| | | | 151°C (K) |
| 6 | -(CH₂)₃ | 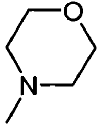 | / |
| | | | (M⁺H)⁺ = 419 |
| | | | 5.67 (C) |
| | | | 91°C (K) |
| 7 | -(CH₂)₂ | 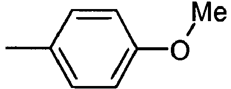 | / |
| | | | (M⁺H)⁺ = 426 |
| | | | 9.69 (C) |
| | | | 180°C (K) |
| 8 | -(CH₂) | 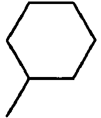 | / |
| | | | (M⁺H)⁺ = 388 |
| | | | 10.45 (C) |
| | | | 148°C (K) |
| 9 | -(CH₂)₂ | 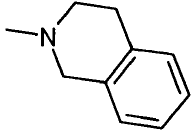 | / |
| | | | (M⁺H)⁺ = 451 |
| | | | 8,19 (C) |
| | | | 144°C (K) |
| 10 | -(CH₂) | 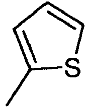 | / |
| | | | (M⁺H)⁺ = 388 |
| | | | 9,36 (C) |
| | | | 116°C (K) |
| 11 | -(CH₂) | 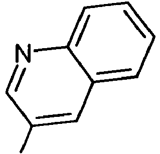 | / |
| | | | (M⁺H)⁺ = 433 |
| | | | 7,09 (C) |
| | | | 180°C (K) |
| 12 | -(CH₂)₂ | 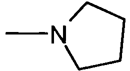 | / |
| | | | (M⁺H)⁺ = 389 |
| | | | 5,69 (C) |
| | | | 87°C (K) |
| 13 | -(CH₂)₂ | 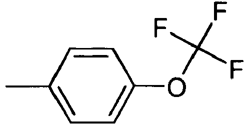 | / |
| | | | (M⁺H)⁺ = 480 |
| | | | 10,47 (C) |
| | | | 150°C (K) |
| 14 | -(CH2)₂ | 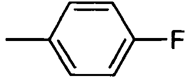 | TFA |
| | | | (M⁻H)⁻ = 412 |
| | | | 1,26 (B) |
| | | | - |
| 15 | -(CH2)₂ | 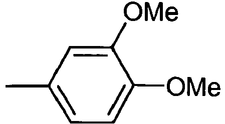 | TFA |
| | | | (M⁻H)⁻ = 454 |
| | | | 1,22 (B) |
| 16 | -(CH2)₂ | 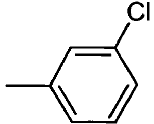 | TFA |
| | | | (M⁻H)⁻ = 428 |
| | | | 1,29 (B) |
| | | | - |
| 17 | -(CH2)₂ | 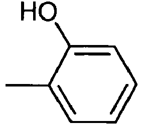 | TFA |
| | | | (M⁻H)⁻ = 410 |
| | | | 1,18 (B) |
| 18 | -(CH2)₂ | 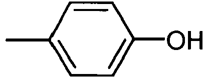 | TFA |
| | | | (M⁻H)⁻ = 410 |
| | | | 1,17 (B) |
| 19 | -(CH₂) | 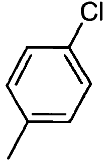 | TFA |
| | | | (M⁻H)⁻ = 414 |
| | | | 1,28 (B) |
| 20 | -(CH₂) | 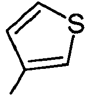 | TFA |
| | | | (M⁻H)⁻ = 386 |
| | | | 1,23 (B) |
| 21 | -(CH₂)₂ | 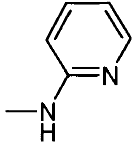 | TFA |
| | | | (M⁻H)⁻ = 412 |
| | | | 0,95 (A) |
| 22 | -(CH₂)₂ | 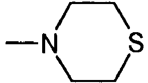 | TFA |
| | | | (M⁺ H)⁺ = 421 |
| | | | 0,93 (A) |
| 23 | -(CH₂) | 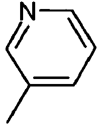 | TFA |
| | | | (M⁺ H)⁺ = 383 |
| | | | 1,00 (A) |
| 24 | -(CH₂) | 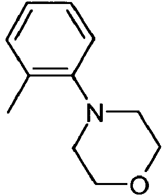 | TFA |
| | | | (M⁺ H)⁺ = 467 |
| | | | 1,27 (A) |
| 25 | -(CH2)₂ | 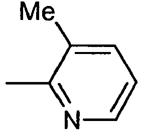 | TFA |
| | | | (M⁺ H)⁺ = 411 |
| | | | 1,00 (A) |
| 26 | -(CH₂) | 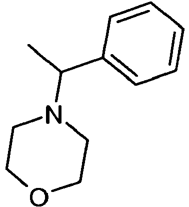 | TFA |
| | | | (M⁺ H)⁺ = 481 |
| | | | 1,05 (A) |
| 27 | -(CH2)₂ | 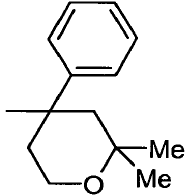 | TFA |
| | | | (M⁺ H)⁺ = 508 |
| | | | 1,31 (A) |
| 28 | -(CH₂)₃ | 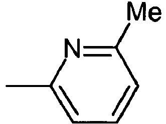 | TFA |
| | | | (M⁺H)⁺ = 411 |
| | | | 0,97 (A) |
| | | | - |
| 29 | -(CH2)₂ | 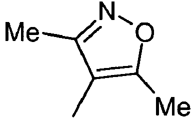 | TFA |
| | | | (M⁺ H)⁺ = 429 |
| | | | 1,20 (A) |
| | | | - |
| 30 | -(CH2)₂ | 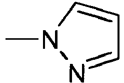 | TFA |
| | | | (M⁺ H)⁺ = 386 |
| | | | 1,13 (A) |
| | | | - |
| 31 | -(CH2)₂ | 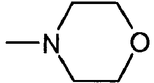 | TFA |
| | | | (M⁺ H)⁺ = 405 |
| | | | 0,90 (A) |
| | | | - |
| 32 | -(CH₂) | 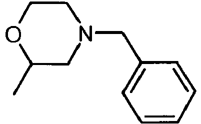 | TFA |
| | | | (M⁺ H)⁺ = 481 |
| | | | 1,00 (A) |
| | | | - |
| 33 | -(CH₂) | 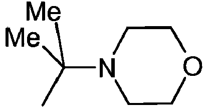 | TFA |
| | | | (M⁺ H)⁺ = 433 |
| | | | 0,94 (A) |
| | | | - |
| 34 | -(CH₂)₃ | 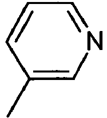 | TFA |
| | | | (M⁺ H)⁺ = 411 |
| | | | 0,98 (A) |
| | | | - |
| 35 | -(CH₂)₂ | 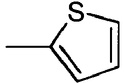 | / |
| | | | (M⁻H)⁻ = 400 |
| | | | 1,25 (B) |
| | | | - |
| 36 | -(CH2)₂ | 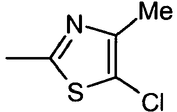 | / |
| | | | (M⁺H)⁺ = 451 |
| | | | 1,27 (A) |
| | | | - |
| 37 | -(CH₂)₃ | 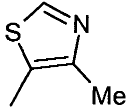 | / |
| | | | (M⁺H)⁺ = 431 |
| | | | 1,17 (A) |
| | | | - |
| 38 | -(CH2)₂ | 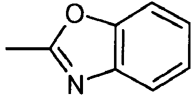 | / |
| | | | (M⁺H)⁺ = 437 |
| | | | 1,22 (A) |
| | | | - |
| 39 | -(CH2)₂ | 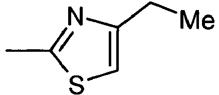 | / |
| | | | (M⁺H)⁺ = 431 |
| | | | 1,23 (A) |
| | | | - |
| 40 | -(CH₂)₂ | 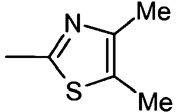 | / |
| | | | (M⁺H)⁺ = 431 |
| | | | 1,21 (A) |
| | | | - |

Les composés répondant à la formule générale (I) objet de l'invention ont fait l'objet d'essais microbiologiques qui ont montré leur intérêt comme substances thérapeutiquement actives.

### Mesure de l'activité inhibitrice des composés selon l'invention vis-à-vis de Mycobacterium tuberculosis.

Le test in-vitro utilisé permet d'identifier des molécules avec une activité antimicrobienne sur la souche de *Mycobacterium tuberculosis* H₃₇Rᵥ. C'est une bactérie de classe 3 de sécurité biologique.

### Matériel et méthode

Le test utilisé est l'Alamar blue (MABA). C'est un test colorimétrique qui permet de déterminer la CMI (concentration minimale inhibitrice) des anti-bactériens. L'Alamar blue est un indicateur d'oxydoréduction passant du bleu au rose en cas de croissance bactérienne. La résazurine (bleue et non fluorescente) est réduite en résorufine (rose et fluorescente) par les bactéries vivantes. La lecture de la plaque est donc visuelle ou par mesure de fluorescence. L'intensité de fluorescence est proportionnelle au nombre de bactéries vivantes.

Ainsi, plus la CMI fluorimétrique a une valeur proche de zéro, moins il faut de produit pour inhiber la croissance totale des bactéries.

Le tableau II illustre les activités de quelques exemples de composés sur la croissance de *Mycobacterium tuberculosis.*

**Tableau II**

| N° | **CMI (nM)** | N° | **CMI (nM)** | N° | **CMI (nM)** | N° | **CMI (nM)** |
|---|---|---|---|---|---|---|---|
| 2 | **224** | 16 | **226** | 25 | **143** | 39 | **154** |
| 7 | **106** | 17 | **151** | 26 | **240** | 40 | **77** |
| 9 | **206** | 18 | **146** | 27 | **272** | | |
| 14 | **270** | 21 | **132** | 36 | **279** | | |
| 15 | **35** | 22 | **156** | 38 | **292** | | |

Ces expériences démontrent que les composés selon la présente invention ont une activité remarquable d'inhibition de la croissance de *M. tuberculosis.* En effet, les antibiotiques standards ont en général des résultats dans le test ci-dessus de l'ordre du micromolaire ou du dizième de micromolaire. Par exemple, la rifampicine inhibe la croissance de *M. tuberculosis* avec une CMI de 0,12 µM et l'isoniazide inhibe la croissance de *M. tuberculosis* avec une CMI de 0,36 µM dans le même test.

Les composés selon l'invention, à savoir les composés répondant à la formule (I) présentent de plus de bonnes propriétés microbiologiques et sont particulièrement aptes à être utilisés pour la préparation de médicament, en particulier d'antibiotique à spectre étroit pour le traitement et/ou la prévention de la tuberculose.

En particulier, ces antibiotiques présentent une action antimicrobienne contre *M. tuberculosis* pour le traitement et/ou la prévention de la tuberculose.

Ainsi, selon un autre de ses aspects, l'invention a pour objet des médicaments qui comprennent un composé de formule (I), ou un sel d'addition de ce dernier à un acide ou à une base pharmaceutiquement acceptable du composé de formule (I).

Ces médicaments trouvent leur emploi en thérapeutique, notamment dans le traitement et/ou la prévention de la tuberculose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Les dits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus ou son sel peut être administré sous forme unitaire d'administration, en mélange avec des excipients pharmaceutiques classiques, aux animaux et aux êtres humains pour la prophylaxie (à adapter au cas par cas) ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

II peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'utilisation des composés de formule (I), ou un de ses sels pharmaceutiquement acceptables ou hydrates ou solvats (à adapter au cas par cas), pour le traitement et/ou la prévention d'infections bactériennes dues à des mycobactéries tels que M. *smegmatis, M. phlei,* ou à d'autres microorganismes tels que *Nocardia brasiliensis, Nocardia absessus* ou *Corynebacterium diphteria,* par exemple.

Ainsi, l'un des aspects de l'invention concerne l'utilisation des composés de formule (I) ou un de ses sels pharmaceutiquement acceptables ou hydrates ou solvats (à adapter au cas par cas), pour le traitement et/ou la prévention des maladies infectieuses telles que la tuberculose, la lèpre, la nocardiose, la diphtérie, l'infection mycobactérienne pulmonaire, l'infection mycobactérienne cutanée, l'infection mycobactérienne atypique et la mycobactériose.

Le terme « tuberculose » comprend les infections dues aux bacilles du complexe tuberculosis *(M. tuberculosis, M. bovis* et M. *africanum*) qui sont tous pathogènes pour l'homme. La tuberculose pulmonaire est de loin la plus fréquente et la plus répandue; il s'agit de la tuberculose du poumon, du larynx, de la trachée et des bronches, la tuberculose des ganglions lymphatiques intrathoraciques, la tuberculose respiratoire de la plèvre, la tuberculose respiratoire primaire et toute autre tuberculose respiratoire. Même si elles sont moins fréquentes, il existe aussi la tuberculose ganglionnaire et la tuberculose extrapulmonaire, la tuberculose du système nerveux tel que la méningite tuberculeuse, la léptomeningite tuberculeuse, les tuberculomes cérébraux et toute autre tuberculose de système nerveux, ou encore la tuberculose osseuse ou articulaire, la tuberculose de système génito-urinaire, la tuberculose périphérique lymphadénopathique, la tuberculose intestinale, péritonéale et/ou des glandes mésentériques, la tuberculose cutanée et des tissus sous-cutanés, la tuberculose de l'oeil, de l'oreille, ou des glandes surrénales, ainsi que la tuberculose disséminée. Le terme « lèpre » (maladie de Hansen) comprend les infections dues à *Mycobacterium leprae* : la lèpre indéterminée, la lèpre tuberculoïde, la lèpre borderline, la lèpre borderline tuberculoïde, la lèpre lépromateuse, ainsi que les autres formes de lèpre.

Le terme « diphtérie » comprend la diphtérie pharyngée, la diphtérie nasopharyngée, la diphtérie cutanée ainsi que les autres formes de diphtérie.

Le terme « nocardiose » comprend la nocardiose pulmonaire, la nocardiose cutanée, et les autres formes de nocardiose.

La présente invention, selon un autre de ses aspects, concerne également un composé de formule (I) pour son utilisation dans une methode de traitement des pathologies ci-dessus indiquées qui comprend l'administration, à un patient, d'une dose efficace d'un composé de formule (I).

## Revendications

1. Composé répondant à la formule (I) : 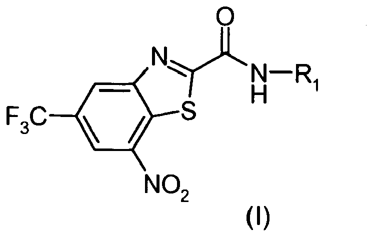 pour lesquels, R₁ représente :
- un C₁₋₄-alkyle substitué par :
. un ou plusieurs atomes d'halogène ;
. un aryle éventuellement substitué par un ou plusieurs groupes choisis indépendamment les uns des autres parmi un groupe OH, C₁₋₃-alcoxy, C₃₋₆-hétérocycloalkyl, C₁-₃-fluoroalcoxy, un atome d'halogène ;
. un hétéroaryle éventuellement substitué par un ou plusieurs groupes choisis indépendamment les uns des autres parmi un groupe C₁₋₄-alkyle, un atome d'halogène ;
. un hétéroaryl-NH non substitué ;
. un C₃₋₆-cycloalkyle non substitué ;
. un C₃₋₆-hétérocycloalkyl éventuellement substitué par un ou plusieurs groupes choisis indépendamment les uns des autres parmi un groupe C₁₋₃-alkyle, aryle et aryl-C₁₋₃-alkyle ;
ou
- un C₁₋₄-alkyle di-substitué par :
. un aryle non substitué ;
et
. un C₃₋₆-hétérocycloalkyle, non substitué ;
à l'état de base ou de sel d'addition à un acide ou à une base.

2. Composé de formule (I) selon la revendication 1, **caractérisé en ce que** R₁ représente :
- un radical C₁₋₄-alkyle substitué par :
. un ou plusieurs atomes de fluor ;
. un phényle éventuellement substitué par un ou plusieurs groupes choisis indépendamment les uns des autres parmi un groupe OH, méthoxy, morpholine, trifluorométhoxy, un atome de chlore ou de fluor ;
. un groupe pyridinyle, isoxazolyle, pyrazolyle, thiényle, benzoxazolyle, tétrahydroisoquinoléinyle, quinoléinyle et thiazolyle éventuellement substitué par un ou plusieurs groupes choisis indépendamment les uns des autres parmi un groupe méthyle, éthyle ou un atome de chlore ;
. un groupe pyridinyl-NH non substitué ;
. un groupe cyclohexyle non substitué ;
. un groupe tétrahydropyranyle, un groupe morpholinyle, un groupe pyrrolidinyle et thiomorpholinyle éventuellement substitué par un ou plusieurs groupes choisis indépendamment les uns des autres parmi un groupe méthyle, phényle et benzyle ;
ou
- un radical C₁₋₄-alkyle di-substitué par :
. un phényle non substitué ;
et
. un groupe morpholinyle non substitué ;
à l'état de base ou de sel d'addition à un acide ou à une base.

3. Composé de formule (I) selon la revendication 1, **caractérisé en ce qu'**il correspond aux composés suivants :
- N-[2-(4-chlorophényl)éthyl]-7-nitro-5-(trifluorométhyl)-1,3-benzothiazole-2-carboxamide ;
- 7-nitro-N-[2-(tétrahydro-2H-pyran-4-yl)éthyl]-5-(trifluorométhyl)-1,3-benzothiazole-2-carboxamide ;
- 7-nitro-N-(2-pyridin-2-yléthyl)-5-(trifluorométhyl)-1,3-benzothiazole-2-carboxamide ;
- 7-nitro-5-(trifluorométhyl)-N-(3,3,3-trifluoropropyl)- 2-benzothiazolecarboxamide ;
- 7-nitro-N-(pyridin-2-ylméthyl)-5-(trifluorométhyl)-1,3-benzothiazole-2-carboxamide ;
- N-[3-(4-morpholinyl)propyl]-7-nitro-5-(trifluorométhyl)-2-benzothiazolecarboxamide ;
- N-[2-(4-méthoxyphényl)éthyl]-7-nitro-5-(trifluorométhyl)-1,3-benzothiazole-2-carboxamide ;
- N-(cyclohexylméthyl)-7-nitro-5-(trifluorométhyl)-1,3-benzothiazole-2-carboxamide ;
- N-[2-(1,2,3,4-tétrahydroisoquinoléin-2(1H)yl)éthyl]-7-nitro-5-(trifluorométhyl)-1,3-benzothiazole-2-carboxamide ;
- 7-nitro-N-(thién-2-ylméthyl)-5-(trifluorométhyl)-1,3-benzothiazole-2-carboxamide ;
- 7-nitro-N-(quinoléin-3-ylméthyl)-5-(trifluorométhyl)-1,3-benzothiazole-2-carboxamide ;
- 7-nitro-N-(2-pyrrolidin-1-yléthyl)-5-(trifluorométhyl)-1,3-benzothiazole-2-carboxamide ;
- 7-nitro-N-{2-[4-(trifluorométhoxy)phényl]éthyl}-5-(trifluorométhyl)-1,3-benzothiazole-2-carboxamide ;
- N-[2-(4-fluorophényl)éthyl]-7-nitro-5-(trifluorométhyl)-1,3-benzothiazole-2-carboxamide ;
- N-[2-(3,4-diméthoxyphényl)éthyl]-7-nitro-5-(trifluorométhyl)-1,3-benzothiazole-2-carboxamide ;
- N-[2-(3-chlorophényl)éthyl]-7-nitro-5-(trifluorométhyl)-1,3-benzothiazole-2-carboxamide ;
- N-[2-(2-hydroxyphényl)éthyl]-7-nitro-5-(trifluorométhyl)-1,3-benzothiazole-2-carboxamide ;
- N-[2-(4-hydroxyphényl)éthyl]-7-nitro-5-(trifluorométhyl)-1,3-benzothiazole-2-carboxamide ;
- N-(4-chlorobenzyl)-7-nitro-5-(trifluorométhyl)-1,3-benzothiazole-2-carboxamide ;
- 7-nitro-N-(thién-3-ylméthyl)-5-(trifluorométhyl)-1,3-benzothiazole-2-carboxamide ;
- 7-nitro-N-[2-(pyridin-2-ylamino)éthyl]-5-(trifluorométhyl)-1,3-benzothiazole-2-carboxamide ;
- 7-nitro-N-(2-thiomorpholin-4-yléthyl)-5-(trifluorométhyl)-1,3-benzothiazole-2-carboxamide ;
- 7-nitro-N-(pyridin-3-ylméthyl)-5-(trifluorométhyl)-1,3-benzothiazole-2-carboxamide ;
- N-(2-morpholin-4-ylbenzyl)-7-nitro-5-(trifluorométhyl)-1,3-benzothiazole-2-carboxamide ;
- N-[2-(3-méthylpyridin-2-yl)éthyl]-7-nitro-5-(trifluorométhyl)-1,3-benzothiazole-2-carboxamide ;
- N-(2-morpholin-4-yl-2-phényléthyl)-7-nitro-5-(trifluorométhyl)-1,3-benzothiazole-2-carboxamide ;
- N-[2-(2,2-diméthyl-4-phényltétrahydro-2H-pyran-4-yl)éthyl]-7-nitro-5-(trifluorométhyl)-1,3-benzothiazole-2-carboxamide ;
- N-[2-(6-méthylpyridin-2-yl)éthyl]-7-nitro-5-(trifluorométhyl)-1,3-benzothiazole-2-carboxamide ;
- N-[3-(3,5-diméthylisoxazol-4-yl)propyl]-7-nitro-5-(trifluorométhyl)-1,3-benzothiazole-2-carboxamide ;
- 7-nitro-N-[2-(1H-pyrazol-1-yl)éthyl]-5-(trifluorométhyl)-1,3-benzothiazole-2-carboxamide ;
- N-(2-morpholin-4-yléthyl)-7-nitro-5-(trifluorométhyl)-1,3-benzothiazole-2-carboxamide ;
- N-[(4-benzylmorpholin-2-yl)méthyl]-7-nitro-5-(trifluorométhyl)-1,3-benzothiazole-2-carboxamide ;
- N-(2-méthyl-2-morpholin-4-ylpropyl)-7-nitro-5-(trifluorométhyl)-1,3-benzothiazole-2-carboxamide ;
- 7-nitro-N-(3-pyridin-3-ylpropyl)-5-(trifluorométhyl)-1,3-benzothiazole-2-carboxamide ;
- 7-nitro-N-(2-thién-2-yléthyl)-5-(trifluorométhyl)-1,3-benzothiazole-2-carboxamide ;
- N-[2-(5-chloro-4-méthyl-1,3-thiazol-2-yl)éthyl]-7-nitro-5-(trifluorométhyl)-1,3-benzothiazole-2-carboxamide ;
- N-[3-(4-méthyl-1,3-thiazol-5-yl)propyl]-7-nitro-5-(trifluorométhyl)-1,3-benzothiazole-2-carboxamide ;
- N-[2-(1,3-benzoxazol-2-yl)éthyl]-7-nitro-5-(trifluorométhyl)-1,3-benzothiazole-2-carboxamide ;
- N-[2-(4-éthyl-1,3-thiazol-2-yl)éthyl]-7-nitro-5-(trifluorométhyl)-1,3-benzothiazole-2-carboxamide ;
- N-[2-(4,5-diméthyl-1,3-thiazol-2-yl)éthyl]-7-nitro-5-(trifluorométhyl)-1,3-benzothiazole-2-carboxamide.

4. Procédé de préparation d'un composé de formule (I) selon l'une quelconque des revendications 1 à 3, **caractérisé en ce que** l'on fait réagir un composé de formule (II) : 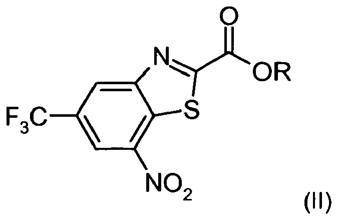 dans laquelle R représente un C₁₋₄alkyle, avec une amine de formule (III) :
H₂N-R₁ (III)
dans laquelle R₁ est tel que défini pour les composés de formule (I).

5. Médicament, **caractérisé en ce qu'**il comprend un composé de formule (I) selon l'une quelconque des revendications 1 à 3, à l'état de base ou de sel d'addition à un acide ou à une base.

6. Composition pharmaceutique, **caractérisée en ce qu'**elle comprend un composé de formule (I) selon l'une quelconque des revendications 1 à 3, à l'état de base ou de sel d'addition à un acide ou à une base, ainsi qu'au moins un excipient pharmaceutiquement acceptable.

7. Composé de formule (I) selon l'une quelconque des revendications 1 à 3 pour son utilisation en tant qu'antibiotique à spectre étroit pour le traitement et/ou la prévention de la tuberculose.

8. Composé de formule (I) pour l'utilisation selon la revendication 7, **caractérisée en ce que** l'antibiotique a une action antimicrobienne pour le traitement et/ou la prévention de la tuberculose.

9. Composé de formule (I) pour l'utlisation selon la revendication 8, **caractérisée en ce que** l'antibiotique a une action antimicrobienne contre *M. tuberculosis.*

## Patentansprüche

1. Verbindung der Formel (I): 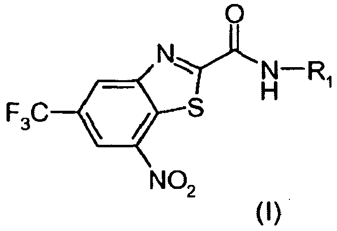 wofür R₁ für:
- ein C₁-C₄-Alkyl, das durch:
. ein oder mehrere Halogenatome;
. ein Aryl, das gegebenenfalls durch eine oder mehrere Gruppen, die unabhängig voneinander aus einer OH-, C₁-C₃-Alkoxy-, C₃-C₆-Heterocycloalkyl- oder C₁-C₃-Fluoralkoxygruppe oder einem Halogenatom ausgewählt sind, substituiert ist;
. ein Heteroaryl, das gegebenenfalls durch eine oder mehrere Gruppen, die unabhängig voneinander aus einer C₁-C₄-Alkylgruppe und einem Halogenatom ausgewählt sind, substituiert ist;
. ein unsubstituiertes Heteroaryl-NH;
. ein unsubstituiertes C₃-C₆-Cycloalkyl;
. ein C₃-C₆-Heterocycyloalkyl, das gegebenenfalls durch eine oder mehrere Gruppen, die unabhängig voneinander aus einer C₁-C₃-Alkyl-, Aryl- und Aryl-C₁-C₃-alkylgruppe ausgewählt sind, substituiert ist;
substituiert ist;
oder
- ein C₁-C₄-Alkyl, das durch:
. ein unsubstituiertes Aryl
und
. ein unsubstituiertes C₃-C₆-Heterocycyloalkyl disubstituiert ist;
steht;
in Basen- oder Säure- oder Basenadditionssalzform.

2. Verbindung der Formel (I) nach Anspruch 1, **dadurch gekennzeichnet, dass** R₁ für:
- einen C₁-C₄-Alkylrest, der durch:
. ein oder mehrere Fluoratome;
. ein Phenyl, das gegebenenfalls durch eine oder mehrere Gruppen, die unabhängig voneinander aus einer OH-Gruppe, Methoxygruppe, Morpholingruppe, Trifluormethoxygruppe, einem Chloratom oder einem Fluoratom ausgewählt sind, substituiert ist;
. eine Pyridinyl-, Isoxazolyl-, Pyrazolyl-, Thienyl-, Benzoxazolyl-, Tetrahydroisochinolinyl-, Chinolinyl- und Thiazolylgruppe, die gegebenenfalls durch eine oder mehrere Gruppen, die unabhängig voneinander aus einer Methylgruppe, einer Ethylgruppe oder einem Chloratom ausgewählt sind, substituiert ist;
. eine unsubstituierte Pyridinyl-NH-Gruppe;
. eine unsubstituierte Cyclohexylgruppe;
. eine Tetrahydropyranylgruppe, eine Morpholinylgruppe, eine Pyrrolidinylgruppe und eine Thiomorpholinylgruppe, die gegebenenfalls durch eine oder mehrere Gruppen, die unabhängig voneinander aus einer Methyl-, Phenyl- und Benzylgruppe ausgewählt sind, substituiert ist;
substituiert ist;
oder
- einen C₁-C₄-Alkylrest, der durch:
. ein unsubstituiertes Phenyl
und
. eine unsubstituierte Morpholinylgruppe disubstituiert ist;
steht;
in Basen- oder Säure- oder Basenadditionssalzform.

3. Verbindung der Formel (I) nach Anspruch 1, **dadurch gekennzeichnet, dass** sie den folgenden Verbindungen entspricht:
- N-[2-(4-Chlorphenyl)ethyl]-7-nitro-5-(trifluormethyl)-1,3-benzothiazol-2-carboxamid;
- 7-Nitro-N-[2-(tetrahydro-2H-pyran-4-yl)ethyl]-5-(trifluormethyl)-1,3-benzothiazol-2-carboxamid;
- 7-Nitro-N-(2-pyridin-2-ylethyl)-5-(trifluormethyl)-1,3-benzothiazol-2-carboxamid;
- 7-Nitro-5-(trifluormethyl)-N-(3,3,3-trifluorpropyl)-2-benzothiazolcarboxamid;
- 7-Nitro-N-(pyridin-2-ylmethyl)-5-(trifluormethyl)-1,3-benzothiazol-2-carboxamid;
- N-[3-(4-Morpholinyl)propyl]-7-nitro-5-(trifluormethyl)-2-benzothiazolcarboxamid;
- N-[2-(4-Methoxyphenyl)ethyl]-7-nitro-5-(trifluormethyl)-1,3-benzothiazol-2-carboxamid;
- N-(Cyclohexylmethyl)-7-nitro-5-(trifluormethyl)-1,3-benzothiazol-2-carboxamid;
- N-[2-(1,2,3,4-Tetrahydroisochinolin-2(1H)-yl)ethyl]-7-nitro-5-(trifluormethyl)-1,3-benzothiazol-2-carboxamid;
- 7-Nitro-N-(thien-2-ylmethyl)-5-(trifluormethyl)-1,3-benzothiazol-2-carboxamid;
- 7-Nitro-N-(chinolin-3-ylmethyl)-5-(trifluormethyl)-1,3-benzothiazol-2-carboxamid;
- 7-Nitro-N-(2-pyrrolidin-1-ylethyl)-5-(trifluormethyl)-1,3-benzothiazol-2-carboxamid;
- 7-Nitro-N-{2-[4-trifluormethoxy)phenyl]ethyl}-5-(trifluormethyl)-1,3-benzothiazol-2-carboxamid;
- N-[2-(4-Fluorphenyl)ethyl]-7-nitro-5-(trifluormethyl)-1,3-benzothiazol-2-carboxamid;
- N-[2-(3,4-Dimethoxyphenyl)ethyl]-7-nitro-5-(trifluormethyl)-1,3-benzothiazol-2-carboxamid;
- N-[2-(3-Chlorphenyl)ethyl]-7-nitro-5-(trifluormethyl)-1,3-benzothiazol-2-carboxamid;
- N-[2-(2-Hydroxyphenyl)ethyl]-7-nitro-5-(trifluormethyl)-1,3-benzothiazol-2-carboxamid;
- N-[2-(4-Hydroxyphenyl)ethyl]-7-nitro-5-(trifluormethyl)-1,3-benzothiazol-2-carboxamid;
- N-(4-Chlorbenzyl)-7-nitro-5-(trifluormethyl)-1,3-benzothiazol-2-carboxamid;
- 7-Nitro-N-(thien-3-ylmethyl)-5-(trifluormethyl)-1,3-benzothiazol-2-carboxamid;
- 7-Nitro-N-[2-(pyridin-2-ylamino)ethyl]-5-(trifluormethyl)-1,3-benzothiazol-2-carboxamid;
- 7-Nitro-N-[2-thiomorpholin-4-ylethyl)-5-(trifluormethyl)-1,3-benzothiazol-2-carboxamid;
- 7-Nitro-N-(pyridin-3-ylmethyl)-5-(trifluormethyl)-1,3-benzothiazol-2-carboxamid;
- N-(2-Morpholin-4-ylbenzyl)-7-nitro-5-(trifluormethyl)-1,3-benzothiazol-2-carboxamid;
- N-[2-(3-Methylpyridin-2-yl)ethyl]-7-nitro-5-(trifluormethyl)-1,3-benzothiazol-2-carboxamid;
- N-(2-Morpholin-4-yl-2-phenylethyl)-7-nitro-5-(trifluormethyl)-1,3-benzothiazol-2-carboxamid;
- N-[2-(2,2-Dimethyl-4-phenyltetrahydro-2H-pyran-4-yl)ethyl]-7-nitro-5-(trifluormethyl)-1,3-benzothiazol-2-carboxamid;
- N-[2-(6-Methylpyridin-2-yl)ethyl]-7-nitro-5-(trifluormethyl)-1,3-benzothiazol-2-carboxamid;
- N-[3-(3,5-Dimethylisoxazol-4-yl)propyl]-7-nitro-5-(trifluormethyl)-1,3-benzothiazol-2-carboxamid;
- 7-Nitro-N-[2-(1H-pyrazol-1-yl)ethyl]-5-(trifluormethyl)-1,3-benzothiazol-2-carboxamid;
- N-(2-Morpholin-4-ylethyl)-7-nitro-5-(trifluormethyl)-1,3-benzothiazol-2-carboxamid;
- N-[(4-Benzylmorpholin-2-yl)methyl]-7-nitro-5-(trifluormethyl)-1,3-benzothiazol-2-carboxamid;
- N-(2-Methyl-2-morpholin-4-ylpropyl)-7-nitro-5-(trifluormethyl)-1,3-benzothiazol-2-carboxamid;
- 7-Nitro-N-(3-pyridin-3-ylpropyl)-5-(trifluormethyl)-1,3-benzothiazol-2-carboxamid;
- 7-Nitro-N-(2-thien-2-ylethyl)-5-(trifluormethyl)-1,3-benzothiazol-2-carboxamid;
- N-[2-(5-Chlor-4-methyl-1,3-thiazol-2-yl)ethyl]-7-nitro-5-(trifluormethyl)-1,3-benzothiazol-2-carboxamid;
- N-[3-(4-Methyl-1,3-thiazol-5-yl)propyl]-7-nitro-5-(trifluormethyl)-1,3-benzothiazol-2-carboxamid;
- N-[2-(1,3-Benzoxazol-2-yl)ethyl]-7-nitro-5-(trifluormethyl)-1,3-benzothiazol-2-carboxamid;
- N-[2-(4-Ethyl-1,3-thiazol-2-yl)ethyl]-7-nitro-5-(trifluormethyl)-1,3-benzothiazol-2-carboxamid;
- N-[2-(4,5-Dimethyl-1,3-thiazol-2-yl)ethyl]-7-nitro-5-(trifluormethyl)-1,3-benzothiazol-2-carboxamid.

4. Verfahren zur Herstellung einer Verbindung der Formel (I) nach einem der Ansprüche 1 bis 3, **dadurch gekennzeichnet, dass** man eine Verbindung der Formel (II): 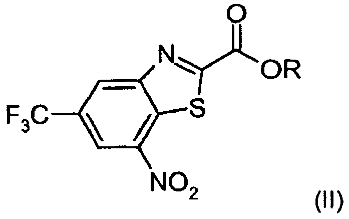 worin R für ein C₁₋₄-Alkyl steht, mit einem Amin der Formel (III):
H₂N-R₁ (III)
worin R₁ wie für die Verbindungen der Formel (I) definiert ist,
umsetzt.

5. Medikament, **dadurch gekennzeichnet, dass** es eine Verbindung der Formel (I) nach einem der Ansprüche 1 bis 3 in Basen- oder Säure- oder Basenadditionssalzform umfasst.

6. Pharmazeutische Zusammensetzung, **dadurch gekennzeichnet, dass** sie eine Verbindung der Formel (I) nach einem der Ansprüche 1 bis 3 in Basen- oder Säure- oder Basenadditionssalzform sowie mindestens einen pharmazeutisch unbedenklichen Hilfsstoff umfasst.

7. Verbindung der Formel (I) nach einem der Ansprüche 1 bis 3 zur Verwendung als Schmalband-Antibiotikum zur Behandlung und/oder Prävention von Tuberkulose.

8. Verbindung der Formel (I) zur Verwendung nach Anspruch 7, **dadurch gekennzeichnet, dass** das Antibiotikum eine antimikrobielle Wirkung aufweist, zur Behandlung und/oder Prävention von Tuberkulose.

9. Verbindung der Formel (I) zur Verwendung nach Anspruch 8, **dadurch gekennzeichnet, dass** das Antibiotikum eine antimikrobielle Wirkung gegen M. *tuberculosis* aufweist.

## Claims

1. Compound corresponding to formula (I): 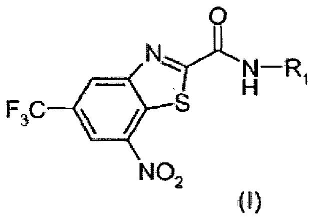 for which, R₁ represents:
- a (C₁-C₄)alkyl substituted with:
. one or more halogen atoms;
. an aryl optionally substituted with one or more groups chosen, independently of one another, from an OH, (C₁-C₃) alkoxy, (C₃-C₆)-heterocycloalkyl or (C₁-C₃)fluoroalkoxy group and a halogen atom;
. a heteroaryl optionally substituted with one or more groups chosen, independently of one another, from a (C₁-C₄) alkyl group and a halogen atom;
. an unsubstituted heteroaryl-NH;
. an unsubstituted (C₃-C₆)cycloalkyl;
. a (C₃-C₆)heterocycloalkyl optionally substituted with one or more groups chosen, independently of one another, from a (C₁-C₃)-alkyl, aryl and aryl (C₁-C₃)alkyl group;
or
- a (C₁-C₄) alkyl disubstituted with:
. an unsubstituted aryl;
and
. an unsubstituted (C₃-C₆)heterocycloalkyl;
in the form of a base or an addition salt with an acid or with a base.

2. Compound of formula (I) according to Claim 1, **characterized in that** R¹ represents:
- a (C₁-C₄)alkyl radical substituted with:
. one or more fluorine atoms;
. a phenyl optionally substituted with one or more groups chosen, independently of one another, from an OH, methoxy, morpholine or trifluoromethoxy group and a chlorine or fluorine atom;
. a pyridinyl, isoxazolyl, pyrazolyl, thienyl, benzoxazolyl, tetrahydroisoquinolinyl, quinolinyl and thiazolyl group optionally substituted with one or more groups chosen, independently of one another, from a methyl or ethyl group or a chlorine atom;
. an unsubstituted pyridinyl-NH group;
. an unsubstituted cyclohexyl group;
. a tetrahydropyranyl group, a morpholinyl group, a pyrrolidinyl group and a thiomorpholinyl group optionally substituted with one or more groups chosen, independently of one another, from a methyl, phenyl and benzyl group;
or
- a (C₁-C₄)alkyl radical disubstituted with:
. an unsubstituted phenyl;
and
. an unsubstituted morpholinyl group;
in the form of a base or of an addition salt with an acid or with a base.

3. Compound of formula (I) according to Claim 1, **characterized in that** it corresponds to the following compounds:
- N-[2-(4-chlorophenyl)ethyl]-7-nitro-5-(trifluoromethyl)-1,3-benzothiazole-2-carboxamide;
- 7-nitro-N-[2-(tetrahydro-2H-pyran-4-yl)ethyl]-5-(trifluoromethyl)-1,3-benzothiazole-2-carboxamide;
- 7-nitro-N-(2-pyridin-2-ylethyl)-5-(trifluoromethyl)-1,3-benzothiazole-2-carboxamide;
- 7-nitro-5-(trifluoromethyl)-N-(3,3,3-trifluoropropyl)-2-benzothiazolecarboxamide;
- 7-nitro-N-(pyridin-2-ylmethyl)-5-(trifluoromethyl)-1,3-benzothiazole-2-carboxamide;
- N-[3-(4-morpholinyl)propyl]-7-nitro-5-(trifluoromethyl)-2-benzothiazolecarboxamide;
- N-[2-(4-methoxyphenyl)ethyl]-7-nitro-5-(trifluoromethyl)-1,3-benzothiazole-2-carboxamide;
- N-(cyclohexylmethyl)-7-nitro-5-(trifluoromethyl)-1,3-benzothiazole-2-carboxamide;
- N-[2-(1,2,3,4-tetrahydroisoquinolin-2(1H)-yl)ethyl]-7-nitro-5-(trifluoromethyl)-1,3-benzothiazole-2-carboxamide;
- 7-nitro-N-(thien-2-ylmethyl)-5-(trifluoromethyl)-1,3-benzothiazole-2-carboxamide;
- 7-nitro-N-(quinolin-3-ylmethyl)-5-(trifluoromethyl)-1,3-benzothiazole-2-carboxamide;
- 7-nitro-N-(2-pyrrolidin-1-ylethyl)-5-(trifluoromethyl)-1,3-benzothiazole-2-carboxamide;
- 7-nitro-N-{2-[4-trifluoromethoxy)phenyl]ethyl}-5-(trifluoromethyl)-1,3-benzothiazole-2-carboxamide;
- N-[2-(4-fluorophenyl)ethyl]-7-nitro-5-(trifluoromethyl)-1,3-benzothiazole-2-carboxamide;
- N-[2-(3,4-dimethoxyphenyl)ethyl]-7-nitro-5-(trifluoromethyl)-1,3-benzothiazole-2-carboxamide;
- N-[2-(3-chlorophenyl)ethyl]-7-nitro-5-(trifluoromethyl)-1,3-benzothiazole-2-carboxamide;
- N-[2-(2-hydroxyphenyl)ethyl]-7-nitro-5-(trifluoromethyl)-1,3-benzothiazole-2-carboxamide;
- N-[2-(4-hydroxyphenyl)ethyl]-7-nitro-5-(trifluoromethyl)-1,3-benzothiazole-2-carboxamide;
- N-(4-chlorobenzyl)-7-nitro-5-(trifluoromethyl)-1,3-benzothiazole-2-carboxamide;
- 7-nitro-N-(thien-3-ylmethyl)-5-(trifluoromethyl)-1,3-benzothiazole-2-carboxamide;
- 7-nitro-N-[2-(pyridin-2-ylamino)ethyl]-5-(trifluoromethyl)-1,3-benzothiazole-2-carboxamide;
- 7-nitro-N-[2-thiomorpholin-4-ylethyl)-5-(trifluoromethyl)-1,3-benzothiazole-2-carboxamide;
- 7-nitro-N-(pyridin-3-ylmethyl)-5-(trifluoromethyl)-1,3-benzothiazole-2-carboxamide;
- N-(2-morpholin-4-ylbenzyl)-7-nitro-5-(trifluoromethyl)-1,3-benzothiazole-2-carboxamide;
- N-[2-(3-methylpyridin-2-yl)ethyl]-7-nitro-5-(trifluoromethyl)-1,3-benzothiazole-2-carboxamide;
- N-(2-morpholin-4-yl-2-phenylethyl)-7-nitro-5-(trifluoromethyl)-1,3-benzothiazole-2-carboxamide;
- N-[2-(2,2-dimethyl-4-phenyltetrahydro-2H-pyran-4-yl)ethyl]-7-nitro-5-(trifluoromethyl)-1,3-benzothiazole-2-carboxamide;
- N-[2-(6-methylpyridin-2-yl)ethyl]-7-nitro-5-(trifluoromethyl)-1,3-benzothiazole-2-carboxamide;
- N-[3-(3,5-dimethylisoxazol-4-yl)propyl]-7-nitro-5-(trifluoromethyl)-1,3-benzothiazole-2-carboxamide;
- 7-nitro-N-[2-(1H-pyrazol-1-yl)ethyl]-5-(trifluoromethyl)-1,3-benzothiazole-2-carboxamide;
- N-(2-morpholin-4-ylethyl)-7-nitro-5-(trifluoromethyl)-1,3-benzothiazole-2-carboxamide;
- N-[(4-benzylmorpholin-2-yl)methyl]-7-nitro-5-(trifluoromethyl)-1,3-benzothiazole-2-carboxamide;
- N-(2-methyl-2-morpholin-4-ylpropyl)-7-nitro-5-(trifluoromethyl)-1,3-benzothiazole-2-carboxamide;
- 7-nitro-N-(3-pyridin-3-ylpropyl)-5-(trifluoromethyl)-1,3-benzothiazole-2-carboxamide;
- 7-nitro-N-(2-thien-2-ylethyl)-5-(trifluoromethyl)-1,3-benzothiazole-2-carboxamide;
- N-[2-(5-chloro-4-methyl-1,3-thiazol-2-yl)ethyl]-7-nitro-5-(trifluoromethyl)-1,3-benzothiazole-2-carboxamide;
- N-[3-(4-methyl-1,3-thiazol-5-yl)propyl]-7-nitro-5-(trifluoromethyl)-1,3-benzothiazole-2-carboxamide;
- N-[2-(1,3-benzoxazol-2-yl)ethyl]-7-nitro-5-(trifluoromethyl)-1,3-benzothiazole-2-carboxamide;
- N-[2-(4-ethyl-1,3-thiazol-2-yl)ethyl]-7-nitro-5-(trifluoromethyl)-1,3-benzothiazole-2-carboxamide;
- N-[2-(4,5-dimethyl-1,3-thiazol-2-yl)ethyl]-7-nitro-5-(trifluoromethyl)-1,3-benzothiazole-2-carboxamide.

4. Process for preparing a compound of formula (I) according to any one of Claims 1 to 3, **characterized in that** a compound of formula (II): 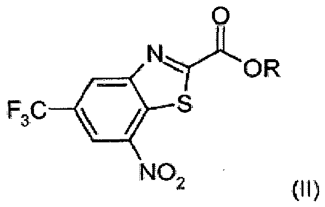 wherein R represents a (C₁-C₄)alkyl, is reacted with an amine of formula (III):
H₂N-R₁ (III)
wherein R₁ is as defined for the compounds of formula (I).

5. Medicament, **characterized in that** it comprises a compound of formula (I) according to any one of Claims 1 to 3, in the form of a base or of an addition salt with an acid or with a base.

6. Pharmaceutical composition, **characterized in that** it comprises a compound of formula (I) according to any one of Claims 1 to 3, in the form of a base or of an addition salt with an acid or with a base, and also at least one pharmaceutically acceptable excipient.

7. Compound of formula (I) according to any one of Claims 1 to 3, for use as a narrow-spectrum antibiotic for the treatment and/or prevention of tuberculosis.

8. Compound of formula (I) for use according to Claim 7, **characterized in that** the antibiotic has an antimicrobial action for the treatment and/or prevention of tuberculosis.

9. Compound of formula (I) for use according to Claim 8, **characterized in that** the antibiotic has an antimicrobial action against M. *tuberculosis.*
